# EUROPEAN PATENT APPLICATION

(11) **EP 4 382 133 A1**
(43) Date of publication of application: **12.06.2024**
(21) Application number: 22853568.8
(22) Date of filing: 05.08.2022
(51) Int. Cl.: A61K 48/00, A61K 31/7088, A61K 47/42, A61P 35/00, C12N 15/113

(54) **PHARMACEUTICAL COMPOSITION FOR TREATING TRIPLE NEGATIVE BREAST CANCER, CONTAINING NON-NATURAL NUCLEIC ACID LIGAND AS ACTIVE INGREDIENT**

(30) Priority: 06.08.2021 KR 20210104105
(71) Applicant: Interoligo Corporation, Gyeonggi-do 14058 (KR)
(72) Inventor: LEE, Jung Hwan, Yongin-si, Gyeonggi-do 15632 (KR); LEE, Jongook, Seongnam-si, Gyeonggi-do 13457 (KR); PARK, Hanseul, Seoul 07026 (KR); LEE, Se Na, Anyang-si, Gyeonggi-do 14016 (KR)
(74) Representative: Kilger, Ute
(86) International application number: PCT/KR2022/011719
(87) International publication number: WO 2023/014193

(57) **Abstract**

One aspect of the present disclosure relates to a pharmaceutical composition for treating triple negative breast cancer, containing, as an active ingredient, a novel non-natural nucleic acid ligand that binds to both human serum albumin (HSA) and a G12D-mutation KRAS protein. The pharmaceutical composition exhibits an excellent effect on the treatment of primary or metastatic triple negative breast cancer, which is difficult to treat. In addition, one aspect of the present disclosure relates to a novel non-natural nucleic acid ligand binding to both human serum albumin (HAS) and a G12D-mutation KRAS protein, and a use thereof. The novel nucleic acid ligand may comprise a nucleic acid sequence of SEQ ID NO: 11 (GNA6AAG6CCGGA6GGCAGC666A6GC) [wherein, the second nucleic acid N is cystidine or gemcitabine and the part represented as 6 is 5-[N-(1-naphthylmethyl) carboxamide]-2'-deoxyuridine (Nap-dU)].

## Description

### TECHNICAL FIELD

The present disclosure relates to a pharmaceutical composition including a novel non-natural nucleic acid ligand as an active ingredient for the treatment of triple-negative breast cancer ("TNBC"). Additionally, the present disclosure pertains to a novel nucleic acid ligand and a use thereof.

### [Description of State-Supported Research and Development]

This application/research was conducted with the support of the National Drug Development Project by the National Drug Development Project Group, funded by the Ministry of Science and ICT, Ministry of Trade, Industry and Energy, and Ministry of Health and Welfare, Republic of Korea (Project Number: HN22C058500).

### BACKGROUND

Breast cancer is the most common cancer among women in advanced countries such as the United States and Europe and has become the leading cause of death for women aged between 40 and 55 in the United States. One in nine women will develop breast cancer during their lifetime, and the number of breast cancer patients is increasing by about 15% each year. In Korea, as of 1995, breast cancer accounted for about 11.9% of female cancer patients, becoming the third most common cancer after cervical and stomach cancer and the fifth most deadly cancer following stomach, liver, cervical, and lung cancer, with its frequency increasing annually.

Triple-negative breast cancer (TNBC) accounts for 10-20% of all breast cancer cases. TNBC is defined by the lack of expression of estrogen receptors (ER) and progesterone receptors (PR) and overexpression or amplification of human epidermal growth factor receptor 2 (HER-2). It is a heterogeneous group of breast cancer tumors diagnosed through immunohistochemistry. TNBC is characterized by aggressive clinical behavior and poor prognosis due to rapid resistance to many chemotherapeutic drugs and a lack of suitable targets. TNBC patients generally have a worse prognosis compared to other breast cancer types, with higher probabilities of early recurrence in distant organs and increased mortality rates. Currently, there are no approved targeted therapies available. Classic microtubule-targeting drugs (MTDs) like paclitaxel and its semi-synthetic derivatives have achieved significant success in clinical control of breast neoplasms. Anthracycline and taxane-based chemotherapy are standard therapies for TNBC. However, most TNBC patients eventually develop drug resistance, tumor relapse, and/or metastasis after temporary responses to initial treatment. Developing drugs to effectively and significantly inhibit the primary or metastatic progression of TNBC, especially metastasis to other organs (notably the lungs), has been challenging. There is an urgent need to develop innovative and more effective therapeutic approaches that achieve more sustained responses in treating TNBC.

Meanwhile, the present inventors have discovered and optimized a novel dual-specific aptamer (nucleic acid ligand) that binds to both Human Serum Albumin (HSA) and the K-RAS G12D mutant protein through SELEX. It was confirmed that this newly developed non-natural nucleic acid ligand has an excellent targeting therapeutic effect on triple-negative breast cancer, particularly when administered intraperitoneally for primary tumor treatment.

Additionally, it is often challenging to completely remove micro-tumors or residual cancer that have deeply infiltrated surrounding tissues after primary breast cancer surgery. Typically, patients do not undergo chemotherapy or radiation therapy for 1-2 weeks post-surgery to allow time for recovery. Until now, there has been no active treatment method for residual or micro-tumors during this period. Therefore, the present disclosure presents the world's first method for actively targeting triple-negative breast cancer during this critical time using the developed pharmaceuticals. This approach is expected to maximize survival rates for patients with triple-negative breast cancer and reduce the risk of recurrence.

### [Patent Literature]

(Patent literature 1) U.S. Patent No. US2008/0318887 A1

### [Non-Patent Literature]

(Non-patent literature 1) Magdalena M. Dailey et al., Nucleic Acids Research, 2010, Vol.38, No. 14, pp. 4877-4888
(Non-patent literature2) YI ZHANG et al. ONCOLOGY LETTERS, 2018, Vol. 15, No.5, pp. 6233-6240
(Non-patent literature3) Park et al., Sci. Transl. Med., 2018, Vol. 10, No.433, eaar1916
(Non-patent literature4) Yoshihisa Tokumaru et al., Am. J. Cancer Res., 2020, Vol. 10, No.3, pp. 897-907

### SUMMARY

The present disclosure aims to provide a pharmaceutical composition containing, as an active ingredient for the treatment of triple-negative breast cancer (TNBC), a novel non-natural nucleic acid ligand that binds to both Human Serum Albumin (HSA) and the K-RAS G12D mutant protein. Also, the present disclosure is to offer a pharmaceutical composition with excellent stability in the body and anticancer effects.

The present disclosure specifically aims to provide a pharmaceutical composition that demonstrates superior efficacy in treating primary or metastatic triple-negative breast cancer, which has been challenging to treat previously. TNBC is characterized by its high malignancy, rapid progression, and swift metastasis. Despite the high demand for targeted therapeutics for TNBC, there has been a lack of suitable targeted therapies since the typical breast cancer targets, estrogen receptor, HER-2 receptor, and progesterone receptor, are negative (no difference compared to normal cells) in TNBC. Additionally, there has been a significant challenge in treating metastatic cancer that appears after surgical resection of TNBC, and no drugs have been available to prevent or treat such metastatic cancer.

The present disclosure aims to provide a novel non-natural nucleic acid ligand that binds to both Human Serum Albumin and the K-RAS G12D mutant protein.

Recent literature has revealed a close association between K-RAS signaling and TNBC (Yoshihisa Tokumaru et al., Am. J. Cancer Res., 2020, Vol. 10, No.3, pp. 897-907).
1. An embodiment of the present disclosure may relate to a pharmaceutical composition for the prevention or treatment of triple-negative breast cancer (TNBC), including a non-natural nucleic acid ligand or a pharmaceutically acceptable salt thereof that binds to human serum albumin (HSA) and K-RAS G12D.
2. An embodiment may pertains to the pharmaceutical composition for prevention or treatment of TNBC, wherein the non-natural synthetic nucleic acid ligand may include the nucleic acid sequence of SEQ ID NO: 11 (GNA6AAG6CCGGA6GGCAGC666A6GC) [wherein the second nucleic acid N represents cysteine or gemcitabine, and the portion marked as "6" represents 5-[N-(1-naphthylmethyl)carboxamide]-2'-deoxyuridine (Nap-dU)].
3. An embodiment may pertains to the pharmaceutical composition for prevention or treatment of TNBC, wherein the pharmaceutical composition includes one or more of the synthetic nucleic acid ligands.
4. An embodiment may pertains to the pharmaceutical composition for prevention or treatment of TNBC, wherein the non-natural nucleic acid ligand comprising the nucleic acid sequence of SEQ ID NO: 11 may be composed of a nucleic acid sequence selected from SEQ ID NOS: 1 to 11.
5. An embodiment may pertains to the pharmaceutical composition for prevention or treatment of TNBC, wherein the TNBC is primary or metastatic triple-negative breast cancer.
6. An embodiment may pertains to the pharmaceutical composition for prevention or treatment of TNBC, wherein the TNBC comprises cancer cells with a K-RAS mutant protein.
7. An embodiment may pertains to the pharmaceutical composition for prevention or treatment of TNBC, wherein the TNBC comprises cancer cells with a K-RAS G12D mutant proteins.
8. An embodiment may pertains to the pharmaceutical composition for prevention or treatment of TNBC, wherein the pharmaceutical composition is administered intravenously, intramuscularly, intraarterially, intraperitoneally, intranasally, intravaginally, intravesically, intradermally, transdermally, topically, or subcutaneously to a subject in need thereof.
9. An embodiment may pertains to the pharmaceutical composition for prevention or treatment of TNBC, wherein the pharmaceutical composition is administered after surgical resection of a triple-negative breast cancer tissue.
10. An embodiment may pertains to the pharmaceutical composition for prevention or treatment of TNBC, wherein, after surgical resection of a triple-negative breast cancer tissue, the pharmaceutical composition is administered into the resected site.
11. An embodiment of the present disclosure may pertain to a non-natural nucleic acid ligand, comprising a nucleic acid sequence selected from SEQ ID NOS: 8 to 11 and binding to human serum albumin (HAS) and K-RAS G12D.
12. An embodiment may pertains to the non-natural nucleic acid ligand that comprises a nucleic acid sequence selected from SEQ ID NOS: 8 to 11.
13. An embodiment of the present disclosure may pertain to a diagnostic composition comprising the non-natural nucleic acid ligand according to an embodiment of the present disclosure.
14. A contrast agent comprising the non-natural nucleic acid ligand according to an embodiment of the present disclosure.
15. An embodiment may pertain to a radiopharmaceutical including the non-natural nucleic acid ligand according to an embodiment of the present disclosure.
16. An embodiment may pertain to a composition for cancer diagnosis, comprising the non-natural nucleic acid ligand according to an embodiment of the present disclosure.
17. An embodiment of the present disclosure may pertain to a method for the prevention or treatment of TNBC, comprising administering, to a subject, a pharmaceutical composition containing the non-natural nucleic acid ligand or pharmaceutically acceptable salt thereof according to an embodiment of the present disclosure, which binds to human serum albumin (HSA) and K-RAS G12D.

The pharmaceutical composition of the present disclosure includes a nucleic acid ligand with specific binding affinities to two or more different targets, thus enabling effective and superior prevention or treatment of primary or metastatic triple-negative breast cancer (TNBC).

The pharmaceutical composition of the present invention, which includes a nucleic acid ligand with specific binding affinities to human serum albumin (HSA) and the K-RAS G12D mutant protein, can effectively inhibit the growth of TNBC tumors.

The pharmaceutical composition of the present disclosure can inhibit metastatic cancer or tumor after the surgical resection of TNBC or excellently suppress the metastasis of cancer or tumor.

With specific binding affinities to human serum albumin (HSA) and the K-RAS G12D mutant protein, the nucleic acid ligand of the present disclosure can excellently target TNBC tumor tissues and/or cells.

The nucleic acid ligand of the present disclosure can inhibit the growth of cancer cells or cause their apoptosis.

By binding a modified nucleotide to a specific sequence of the oligonucleotide thereof, the nucleic acid ligand of the present disclosure can reduce the rate of degradation by in vivo enzymes.

### BRIEF DESCRIPTION OF DRAWINGS

FIG. 1 shows a schematic flowchart for obtaining single nucleic acid ligands by using a plasma protein as a first target protein and a cancer cell-specific protein as a second target protein according to one embodiment.
FIG. 2 is a schematic diagram illustrating a selection method, which is a method for preparing a nucleic acid ligand according to one embodiment.
FIG. 3 shows the results of bead-based ELISA binding assay in which the binding affinity of nucleic acid library pools obtained through primary SELEX for a plasma protein (human serum albumin) was analyzed.
FIG. 4 shows the results of filter binding assay in which the binding affinity of nucleic acid library pools obtained through primary SELEX for a plasma protein (albumin) was analyzed.
FIG. 5 shows the results of analyzing binding affinity of nucleic acid library pools obtained through secondary SELEX for K-ARS G12D mutant protein.
FIG. 6A shows the results of analyzing binding affinity of clone nucleic acid ligands selected from the secondary SELEX nucleic acid library pools for human serum albumin (HSA).
FIG. 6B shows the results of analyzing binding affinity of clone nucleic acid ligands selected from the secondary SELEX nucleic acid library pools for K-RAS G12D protein.

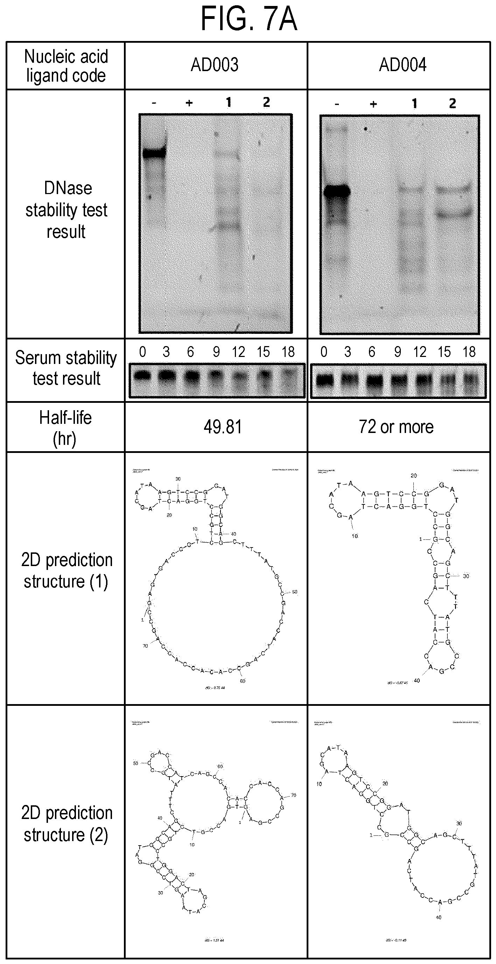

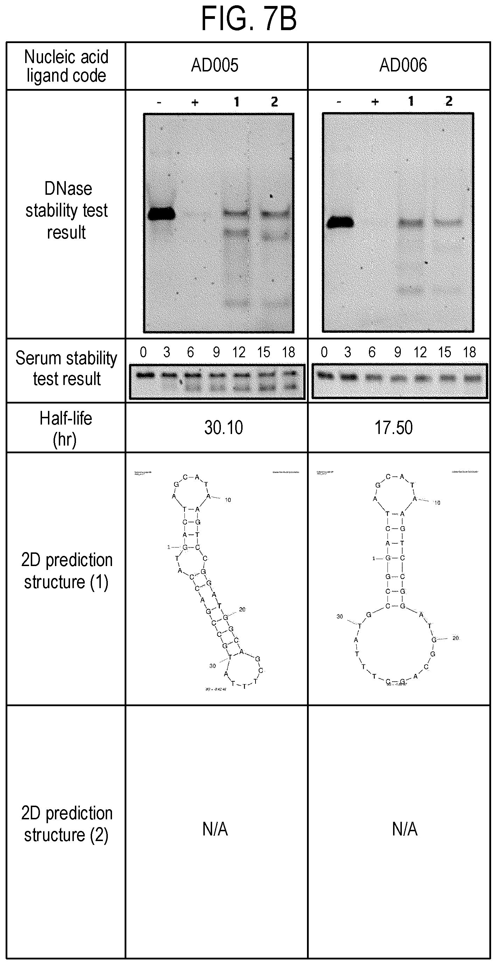

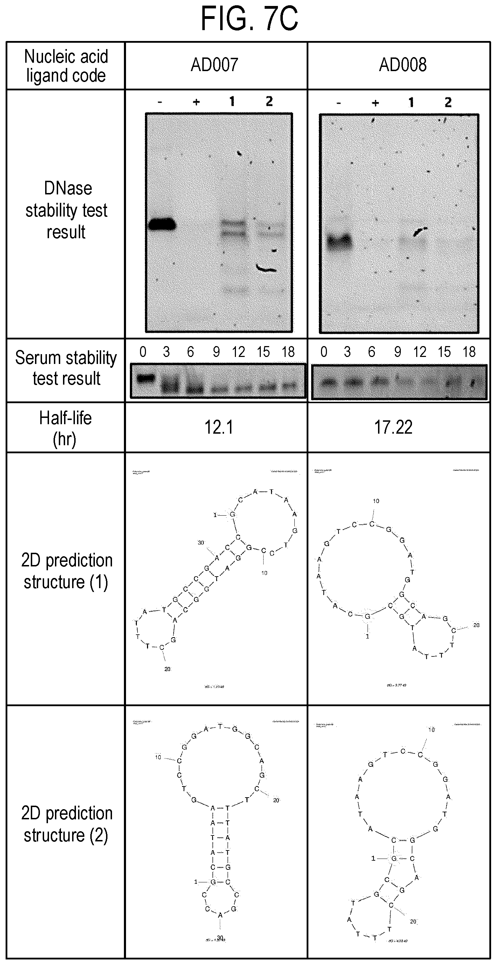

FIGS. 7A to 7C show DNase stability results of human serum albumin (HSA)- and K-RAS G12D-targeted nucleic acid ligands according to one embodiment against human serum albumin (HSA) and K-RAS G12D protein and 2D prediction structures of the individual nucleic acid ligands. In the DNase stability test results, the sign (-) indicates a negative control without the addition of target proteins and a nuclease; the sign (+) indicates a positive control with the addition of only a nuclease; the numeral 1 indicates a test group with human serum albumin and a nuclease, and the numeral 2 indicates a test group with K-RAS G12D protein and a nuclease.
FIG. 8 shows the UPLC analysis results, before and after purification, of the nucleic acid ligand AD005 according to one embodiment.
FIGS. 9A to 9E show the molecular weight analysis results of the nucleic acid ligands according to an embodiment after purification of nucleic acid ligand AD005 in FIG. 9A, nucleic acid ligand AD089 in FIG. 9B, nucleic acid ligand AD210 in FIG. 9C, nucleic acid ligand AD211 in FIG. 9D, and nucleic acid ligand AD212 in FIG. 9E.
FIG. 10 is a schematic diagram illustrating an experimental overview of evaluating the anticancer efficacy of a pharmaceutical composition containing the nucleic acid ligand AD005 according to an embodiment in a triple-negative breast cancer model (4TI TNBC mouse model).
FIG. 11 presents the results of an experiment measuring tumor size after administering varying doses of a pharmaceutical composition containing the nucleic acid ligand AD005 according to an embodiment in a triple-negative breast cancer model (4TI TNBC mouse model) to evaluate the anticancer efficacy.
FIG. 12 is a schematic diagram outlining the experimental approach to assess the efficacy of the pharmaceutical composition containing the nucleic acid ligand AD005 in inhibiting metastasis and recurrence in a TNBC orthotopic mouse model (tumor resection model).
FIG. 13 shows the results of experiments assessing the efficacy of the pharmaceutical composition containing the nucleic acid ligand AD005 in inhibiting metastasis and recurrence in the TNBC orthotopic mouse model (tumor resection model).
FIG. 14 is a schematic diagram outlining the experimental approach to assess the efficacy of the pharmaceutical compositions containing nucleic acid ligands AD005 (300µg or 600µg) and AD089 (300µg or 600µg) in inhibiting metastasis and recurrence in a TNBC orthotopic mouse model (tumor resection model).
FIG. 15 presents the results of experiments assessing the efficacy of the pharmaceutical compositions containing nucleic acid ligands AD005 (300µg or 600µg) and AD089 (300µg or 600µg) in inhibiting metastasis and recurrence in the TNBC orthotopic mouse model (tumor resection model).
FIGS. 16A and 16B show survival plots based on the experimental results assessing the efficacy of nucleic acid ligands AD005 (300µg or 600µg) and AD089 (300µg or 600µg) in the TNBC orthotopic mouse model (tumor resection model). FIG. 16A is a survival plot for the administration of AD005 (300µg or 600µg), and FIG. 16B is a survival plot for AD089 (300µg or 600µg). The data excludes individuals where the primary tumor was not completely removed during surgery.
FIG. 17 is a schematic diagram outlining the experimental approach to assess the efficacy of the pharmaceutical compositions containing nucleic acid ligands AD210 to AD212 in inhibiting metastasis and recurrence in the TNBC orthotopic mouse model (tumor resection model).
FIG. 18 shows the results of experiments assessing the efficacy of the pharmaceutical compositions containing nucleic acid ligands AD210 to AD212 (each at 100µg) in inhibiting metastasis and recurrence in the TNBC orthotopic mouse model (tumor resection model).

### DETAILED DESCRIPTION

Various embodiments described herein are illustrated for the purpose of clarifying the technical idea of the present disclosure, and are not intended to limit the present disclosure to any specific embodiment. The technical idea of the present disclosure includes various modifications, equivalents, alternatives and embodiments selectively combined from all or part of individual embodiments described in the present document. Further, the scope of the technical idea of the present disclosure is not limited to the various embodiments described below, and the detailed description thereof.

The terms used herein, including technical or scientific terms, may have meanings that are generally understood by a person having ordinary knowledge in the art to which the present disclosure pertains, unless otherwise specified. It will be further understood that terms, such as those defined in commonly used dictionaries, should be interpreted as having a meaning that is consistent with their meaning in the context of the relevant art and the present disclosure, and will not be interpreted in an idealized or overly formal sense unless expressly so defined herein.

The singular of an expression used herein may include the meaning of the plural of the expression unless otherwise indicated in the context clearly dictates otherwise, and the same applies to singular forms of expressions as set forth in the claims.

The expressions "1st", "2nd", "first", "second", and the like used herein are used to distinguish one object from another in referring to plural same objects unless otherwise indicated in the context, and do not limit the order or importance of the objects.

The expressions used herein, "A, B and C", "A, B or C", "A, B and/or C", "at least one of A, B, and C", "at least one of A, B, or C", "at least one of A, B, and/or C", "at least one selected from A, B, and C", "at least one selected from A, B, or C", "at least one selected from A, B, and/C", and the like may be used to refer to each listed item or any possible combination of the listed items may be provided. For example, the expression "at least one selected from A and B" may refer to all of (1) A, (2) at least one of A, (3) B, (4) at least one of B, (5) at least one of A and at least one of B, (6) B and at least one of A, (7) A and at least one of B, and (8) both of A and B.

The term "about" or "approximately" used herein may refer to the usual error range for each value, as is widely known to a person skilled in the art. In the context of a numerical value or range described herein, the term may mean ±20%, ±15%, ±10%, ±9%, ±8%, ±7%, ±6%, ±5%, ±4%, ±3%, ±2%, or ±1% of a numerical value or range recited or claimed in an embodiment.

As used herein, the terms "SELEX", "SELEX technology", "SELEX method", "SELEX process" may be used interchangeably with each other, and may refer to a combined method of a process of selecting a nucleic acid interacting with a target in a preferred manner (e.g., binding to a protein) and a process of amplifying the selected nucleic acid. This SELEX method is an in vitro method for selecting a nucleic acid molecule capable of binding to a target molecule with high specificity, which is disclosed in U.S. Pat. No. 5,475,096 ("Nucleic Acid Ligands") and U.S. Pat. No. 5,270,163 (WO 91/19813, "Nucleic Acid Ligands").

The present disclosure provides a pharmaceutical composition including the nucleic acid ligand or a pharmaceutically acceptable salt thereof described above for treating triple-negative breast cancer.

An embodiment is concerned with a novel non-natural nucleic acid ligand, which is a new class of nucleic acid compound that was previously considered impossible to exist.

As used herein, the terms "novel nucleic acid ligand", "nucleic acid ligand", "unnatural nucleic acid ligand", and "novel unnatural nucleic acid ligand" may be used interchangeably with one another. These terms may refer to a nucleic acid molecule having specific binding affinities for two or more different targets.

As used herein, the term "non-natural" (synthetic or unnatural) refers to non-occurring in nature, and in this regard, the novel nucleic acid ligand according to one embodiment of the present disclosure may not be a nucleic acid with a known physiological function binding to a target.

In one embodiment, the novel nucleic acid ligand may have specific binding affinities for two or more different targets. For example, the novel nucleic acid ligand may have specific binding affinities for two, three, four, five, six, or more targets.

In one embodiment, the novel nucleic acid ligand may have specific binding affinities for two different targets.

As used herein, the term "specific binding affinity" may mean that the nucleic acid ligand binds to its target with generally much higher affinity than binding to other non-target components or ingredients present in a mixture or sample. In one embodiment, a plurality of nucleic acid ligand candidate sequences may be selected from a nucleic acid library or a nucleic acid mixture having specific binding affinities for two or more different targets, and the novel nucleic acid ligand according to one embodiment may be selected by confirmation to have specific binding affinities for two or more different targets from among the selected plurality of nucleic acid ligand candidate sequences.

In an embodiment, the targets may have a three-dimensional structure. In an embodiment, the targets do not include a polynucleotide that binds to a nucleic acid through a mechanism which predominantly depends on Watson/Crick base pairing or triple helix binding.

In an embodiment, the novel nucleic acid ligand may interact with the targets by three-dimensionally binding to the targets, recognizing three-dimensional structures of the targets, or forming three-dimensional structures by itself. The novel nucleic acid ligand may regulate characteristics of respective targets by binding to the targets, recognizing three-dimensional structures of the targets, or interacting with the targets. For example, a novel nucleic acid ligand according to an example can bind to a target protein to inhibit the activity of a protein, block signaling pathways, or inhibit activity of proteins downstream of signaling pathways.

As used herein, the term "binding" or "interacting" may refer to the association between two molecules (e.g., a stable association between a nucleic acid ligand and a target) due to, for example, pi-stacking, electrostatic interactions, hydrophobic interactions, ionic interactions, and/or hydrogen bonding interactions, under physiological conditions.

As used herein, the term "regulating" may refer to changing functional characteristics, biological activity, and/or biological mechanisms (or pathways) of targets. For example, the term may refer to upregulating (e.g., activating or stimulating), downregulating (e.g., inhibiting or suppressing), or changing characteristics, activity, and/or mechanisms of targets. By way of example, when the target is a cell, the characteristics of the cell to be regulated may be cell viability, cell proliferation, gene expression, cell morphology, cell activation, phosphorylation, calcium mobilization, degranulation, cell migration, and/or cell differentiation.

In one embodiment, the novel nucleic acid ligand is neither a conventional aptamer that binds to only one specific target, nor a bispecific aptamer obtained by coupling or connecting a plurality of different aptamers to each other.

In one embodiment, the novel nucleic acid ligand is not in the form of a coupled body of a plurality of aptamers (non-coupling).

As used herein, the term "coupled body" may refer to a body obtained by coupling, fusing, linking, or conjugating two or more separate aptamers. For example, the coupled body may mean a dimer, a trimer, a tetramer, or a higher-order multimer of any monomeric aptamers, or may mean a bispecific aptamer obtained by coupling or linking two or more different aptamers, or may mean a complex/conjugate/linkage composed of a plurality of aptamers.

In one embodiment, the novel nucleic acid ligand may be designed such that all or a part of its nucleic acid sequence forming a binding site to one target may form all or a part of the nucleic acid sequence forming a binding site to another target. In one embodiment, the nucleic acid sequence forming a binding site to a target may include a sequence of a 5' or 3' primer for amplification of a nucleic acid ligand or a part of the sequence.

In one embodiment, the part of the nucleic acid sequence forming a binding site to a target may be about 1% to about 99%, about 5% to about 95%, about 10% to about 90%, about 20% to about 80%, about 30% to about 70%, or about 40% to about 60% of the entire sequence of the nucleic acid ligand, but is not limited thereto. In one embodiment, the part of the nucleic acid sequence forming a binding site to a target may contain one or more nucleotides constituting the nucleic acid ligand, but is not limited thereto.

In one embodiment, the novel nucleic acid ligand may be a nucleic acid ligand in which binding sites to two or more targets are not present separately from each other. For example, a nucleic acid ligand in which the binding sites to targets are present separately from each other may mean an aptamer multimer, a bispecific aptamer, or a complex/conjugate/linkage composed of a plurality of aptamers.

In one embodiment, in the novel nucleic acid ligand, binding sites to two or more targets may be formed in one single nucleic acid ligand or from one single nucleic acid ligand. This may mean that tertiary structures of the nucleic acid ligand, which are formed to bind to the targets, are not formed from separate sequences, respectively, or formed by coupling ones formed from separate sequences, but formed from one nucleic acid ligand or the sequence of a single nucleic acid ligand. Therefore, the novel nucleic acid ligand corresponds to a substance that is different or distinguished from a bi-specific aptamer.

In an embodiment, the novel nucleic acid ligand may consist of about 100 or less nucleotides. Specifically, the novel nucleic acid ligand may consist of 100 or less, 90 or less, 80 or less, 70 or less, 60 or less, 50 or less, or 40 or less nucleotides. In an embodiment, the novel nucleic acid ligand may consist of 10 to 105, 15 to 100, 20 to 95, 25 to 90, 30 to 85, 35 to 80, 40 to 75, 45 to 70, 50 to 65, or 55 to 60 nucleotides.

In an embodiment, the nucleotides may be selected from the group consisting of DNA nucleotides, RNA nucleotides, modified nucleotides thereof, and combinations thereof.

The oligonucleotide of the present disclosure can selectively bind to cancer cells and inhibit their growth through various mechanisms within the cells.

By attaching one or more modified nucleotides to the oligonucleotide of the present disclosure, the modified nucleotide can be stabilized or prevented from being inactivated in the body.

Furthermore, the modified nucleotide can be inserted, substituted, or attached at any position in the oligonucleotide sequence. This modified nucleotide could be one with anticancer effects (e.g., gemcitabine). The modified nucleotide can be a chemically modified nucleoside or nucleotide.

The modified nucleotide may be linked to the 5' end, 3' end or any one or more nucleotides located in the oligonucleotide, with preference for the 5' end of the oligonucleotide.

As used herein, the term "modified nucleotide" may refer to an analog, substituent, or ester of a naturally occurring nucleotide (A, G, T/U, and C), and may mean a broad concept encompassing modified nucleotides easily available by a person skilled in the art. For example, the modified nucleotide may refer to a nucleotide having a substitution at the C-5 position (e.g., a C-5 modified pyrimidine), a nucleotide having a modified sugar (ribose or deoxyribose) constituting the nucleotide, or a nucleotide having all of such modifications.

In an embodiment, the C-5 modified pyrimidine may refer to a C-5 modified amino carbonyl pyrimidine, and examples thereof may include 5-(N-benzylcarboxyamide)-2'-deoxyuridine (Bz-dU), 5-(N-isobutylcarboxyamide)-2'-deoxyuridine (iBu-dU), 5-(N-phenethylcarboxyamide)-2'-deoxyuridine (Pe-dU), 5-(N-thiophenylmethylcarboxyamide)-2'-deoxyuridine (Th-dU), 5-(N-[2-(1H-indol-3y1)ethyl]carboxyamide)-2'-deoxyuridine (Trp-dU), 5-(N-[1-(3-trimethylammonium)propyl]carboxyamide)-2'-deoxyuridine chloride, 5-(N-naphthylmethylcarboxyamide)-2'-deoxyuridine (Nap-dU), 5-(N-[1-(2,3-dihydroxypropyl)]carboxyamide)-2'-deoxyuridine), 5-(N-benzylcarboxyamide)-2'-deoxycytidine (Bz-dC), 5-(N-isobutylcarboxyamide)-2'-deoxycytidine (iBu-dC), 5-(N-phenethylcarboxyamide)-2'-deoxycytidine (Pe-dC), 5-(N-thiophenylmethylcarboxyamide)-2'-deoxycytidine (Th-dC), 5-(N-[2-(1H-indol-3y1)ethyl]carboxyamide)-2'-deoxycytidine (Trp-dC), 5-(N-[1-(3-trimethylammonium)propyl]carboxyamide)-2'-deoxycytidine chloride, 5-(N-naphthylmethylcarboxyamide)-2'-deoxycytidine (Nap-dC), and/or 5-(N-[1-(2,3-dihydroxypropyl)]carboxyamide)-2'-deoxycytidine, but is not limited thereto.

In an embodiment, a modified nucleotide having a 2'-position modification of the ribose sugar may include 2'-deoxy-2'-fluorouridine (Uf), 2'-deoxy-2'-fluorocytidine (Cf), 2'-hydroxyadenosine (Ar), 2'-methoxyadenosine (Am), and 2'-methoxyguanosine (Gm), but with no limitations thereto.

In an embodiment, a modified nucleotide having both a C-5 modification and a 2' position modification of the ribose sugar may include 5-(N-benzylcarboxyamide)-2'-O-methyluridine, 5-(N-benzylcarboxyamide)-2'-fluorouridine, 5-(N-isobutylcarboxyamide)-2'-O-methyluridine, 5-(N-isobutylcarboxyamide)-2'-fluorouridine, 5-(N-[2-(1H-indol-3yl)ethyl]carboxyamide)-2'-O-methyluridine, 5-(N-[2-(1H-indol-3yl)ethyl]carboxyamide)-2'-O-fluorouridine, 5-(N-naphthylmethylcarboxyamide)-2'-O-methyluridine, 5-(N-naphthylmethylcarboxyamide)-2'-fluorouridine, 5-(N-[1-(2,3-dihydroxypropyl)]carboxyamide)-2'-O-methyluridine, and/or 5-(N-[1-(2,3-dihydroxypropyl)]carboxyamide)-2'-O-fluorouridine, but with no limitations thereto.

In an embodiment, the modified nucleotide having both a C-5 modification and a 2' position modification of the ribose sugar may include 5-(N-benzylcarboxyamide)-2'-O-methylcytidine, 5-(N-benzylcarboxyamide)-2'-fluorocytidine, 5-(N-isobutylcarboxyamide)-2'-O-methylcytidine, 5-(N-isobutylcarboxyamide)-2'-fluorocytidine, 5-(N-[2-(1H-indol-3yl)ethyl]carboxyamide)-2'-O-methylcytidine, 5-(N-[2-(1H-indol-3y1)ethyl]carboxyamide)-2'-O-fluorocytidine, 5-(N-naphthylmethylcarboxyamide)-2'-O-methylcytidine, 5-(N-naphthylmethylcarboxyamide)-2'-fluorocytidine, 5-(N-[1-(2,3-dihydroxypropyl)]carboxyamide)-2'-O-methylcytidine, and/or 5-(N-[1-(2,3-dihydroxypropyl)]carboxyamide)-2'-O-fluorocytidine, but is not limited thereto.

In an embodiment, the modified nucleotide may be 5-(N-benzylcarboxyamide)-2'-deoxyuridine (Bz-dU) or 5-(N-naphthylmethylcarboxyamide)-2'-deoxyuridine (Nap-dU). Thus, at least one of the nucleotides constituting the novel nucleic acid ligand may be such a modified nucleotide.

When modified nucleotides are attached to the 3' end of an oligonucleotide, additional elements such as inverted dT (idT), locked nucleic acid (LNA), polyethylene glycol (PEG), or 2'-methoxy nucleosides (2'OMeNu) may be linked to the 3' end of the modified nucleotides.

The additional element idT, LNA, PEG, or 2'OMeNu linked to the 3' end of the modified nucleotide protects the 3' end of the oligonucleotide from nucleases and reduces the degradation of the oligonucleotide in the body, allowing the modified nucleotide to remain attached to the oligonucleotide for a longer duration, potentially enhancing the anticancer efficacy thereof.

In an embodiment, the novel nucleic acid ligand may include one nucleic acid sequence selected from the group consisting of SEQ ID NO: 1 to SEQ ID NO: 11.

In an embodiment, the novel nucleic acid ligand may be selected from the group consisting of single-stranded RNA, double-stranded RNA, single-stranded DNA, and double-stranded DNA.

In an embodiment, the novel nucleic acid ligand may be used by conjugating biotin in front of the 5'-end or the back of the 3'-end as needed.

In an embodiment, the novel nucleic acid ligand may be used by conjugating a fluorescent substance (e.g., FAM, VIC, HEX, BHQ-1, Cy5, Cy3, rhodamine, Texas Red, etc.) in front of the 5'-end or the back of the 3'-end as needed.

An embodiment of the present disclosure may pertains to a non-natural nucleic acid ligand manufactured by the method according to an embodiment.

To connect oligonucleotides and modified nucleotides, one method may include a step of inserting, substituting, or attaching the modified nucleotide at any position in the sequence of the aforementioned oligonucleotide or attaching the modified nucleotide to at least one of the 5' and 3' ends of the aforementioned oligonucleotide.

Each nucleotide in the oligonucleotide sequence of the present disclosure can be substituted with a modified nucleic acid or modified nucleotide, and the oligonucleotide sequence may contain one or more modified nucleic acids or nucleotides therein.

The oligonucleotide of the present disclosure could be a nucleic acid ligand with specific binding affinity for two or more different targets. Such nucleic acid ligands are described in detail in Korean Patent Application No. 10-2021-0059607 issued to the present inventors. The nucleic acid ligands are shown in Table 1. In Table 1, the portion marked as "6" represents 5-[N-(1-naphthylmethyl)carboxamide]-2'-deoxyuridine (Nap-dU) and the portion marked as "9" represents gemcitabine. Furthermore, the nucleic acid ligands below are composed of DNA, which consists of deoxyribose-based nucleic acids, where G stands for 2'-deoxyguanosine, C for 2'-deoxycytidine, A for 2'-deoxyadenosine, and T for thymidine.

**TABLE 1**

| Nucleic acid ligand code | Base sequence |
|---|---|
| AD003 | GAGTGACCGTCTGCCTGGAC6AGCA6AAG6CCGGA6GGCAG C666A6GCCGACCA6CAGCCACACCACCAGCC (SEQ ID NO:: 1) |
| AD004 | GCCTGGAC6AGCA6AAG6CCGGA6GGCAGC666A6GCCGACC A6CAGCC (SEQ ID NO:: 2) |
| **AD005** | **GAC6AGCA6AAG6CCGGA6GGCAGC666A6GCCGACCA6** (SEQ ID NO:: 3) |
| AD006 | GAC6AGCA6AAG6CCGGA6GGCAGC666A6GCCG (SEQ ID NO:: 4) |
| AD007 | GCA6AAG6CCGGA6GGCAGC666A6GCCGACC (SEQ ID NO:: 5) |
| AD008 | GCA6AAG6CCGGA6GGCAGC666A6GC (SEQ ID NO:: 6) |
| AD089 | GAC6AGCA6AAG6CCGGA6GGCAGC666A6GCC (SEQ ID NO:: 7) |
| AD210 | 99TGGAC6AGCA6AAG6CCGGA6GGCAGC666A6GCCGACCA6 (SEQ ID NO:: 8) |
| AD211 | GAC6AGCA6AAG6CCGGA6GGCAGC666A6GCCGA99A6 (SEQ ID NO:: 9) |
| AD212 | GA96AG9A6AAG6CCGGA6GGCAGC666A6GCCGACCA6 (SEQ ID NO:: 10) |
| Common sequence | GNA6AAG6CCGGA6GGCAGC666A6GC (SEQ ID NO:: 11) |

In an embodiment, the pharmaceutical composition may be a drug delivery composition. In a pharmaceutical composition according to an example of the present disclosure, a novel nucleic acid ligand may be delivered into cancer cells having a therapeutic target via a pathway of endocytosis or the like. The novel nucleic acid ligand may bind to a plasma protein present in the body of a subject to which the pharmaceutical composition is administered. Therefore, the pharmaceutical composition containing the novel nucleic acid ligand can exhibit excellent in vivo stability and slow renal clearance (excellent residence time in the body), and the novel nucleic acid ligand can enter cancer cells via a pathway of endocytosis or the like after reaching the cancer tissue.

As used herein, the term "subject" refers to mammals including humans, such as horses, sheep, pigs, goats, dogs, and the like, that have or may develop a disease or a disorder caused by a target or its action, with preference for humans.

As used herein, the term "endocytosis" refers to a cellular action by which a cell transports a substance outside the cell membrane into the cell membrane. By endocytosis, a foreign substance is sent into the cell while enclosed in the cell membrane, and the absorbed substance is degraded by vesicle formation in the cell and then recycled or discharged to the outside. Endocytosis may be divided into macropinocytosis, receptor-mediated endocytosis involving clathrin protein, caveolae endocytosis, and the like, depending on the method of activation or the type of protein involved.

In an embodiment, the pharmaceutical composition may further contain a plasma protein. In this regard, the pharmaceutical composition may be in the form of a conjugate in which the plasma protein is bound to the novel nucleic acid ligand or in the form of the novel nucleic acid ligand alone, and the plasma protein may be a protein that is present outside the body of a subject to be treated.

In a pharmaceutical composition according to an example of the present disclosure, a novel nucleic acid ligand may be delivered into cancer cells having a therapeutic target via a pathway of macropinocytosis or the like.

As used herein, the term "macropinocytosis" is one type of endocytosis, wherein a pocket may be formed while the cellular membrane protrudes out of a cell and then contracts again by the cytoskeletal protein actin and the formed pocket may be separated while entering the cell, thereby forming vesicles (macropinosomes) with a diameter of about 0.2 µm to 5 µm.

Macropinocytosis plays an important role in tumor metabolism, which recently attracts attention. Through macropinocytosis, nutrients (e.g., glutamine, albumin, etc.) necessary for rapid growth of tumor cells may be supplied into cancer cells and used for metabolism. That is, extracellular fluid, extracellular molecules, and the like may be contained inside the vesicles formed by macropinocytosis. Macropinocytosis may be activated by mutation of a tumor gene or protein of cancer cells. For example, the tumor gene may be SRC gene or K-RAS gene. For example, macropinocytosis may be activated by mutation of KRAS gene or protein of cancer cells. The mutation of K-RAS protein may be caused by mutations of the 12th, 13th, and 61st amino acids of the wild-type K-RAS protein, and for example, may be K-RAS G12D, K-RAS G12V, K-RAS G12C, K-RAS G12A, K-RAS G12S, K-RAS G12R, K-RAS G13D, or K-RAS Q61H.

In one embodiment, the efficiency of delivery of the nucleic acid ligand into the cell by macropinocytosis can be increased by binding of the nucleic acid ligand and the plasma protein. That is, the nucleic acid ligand binding to the plasma protein may be excellent in the delivery into cells compared with nucleic acid ligands not binding to the plasma protein.

A nucleic acid ligand according to one embodiment of the present disclosure can be delivered into the cytoplasm through various mechanisms of endocytosis. Macropinocytosis, which has attracted attention as a main pathway for supplying essential nutrients necessary for rapid proliferation of cancer cells, as well as other endocytosis pathways may be involved in the delivery of the nucleic acid ligand. The nucleic acid ligand delivered into the cytoplasm can inhibit the proliferation of cancer cells by binding to a therapeutic target protein to disturb signaling pathways.

In one embodiment, the nucleic acid ligand delivered into cells can bind to a K-RAS mutant protein (e.g., K-RAS G12D) inside cancer cells to inhibit the phosphorylation of proteins (ERK, etc.) downstream of the K-RAS signaling pathways, thereby inhibiting excessive cell growth, proliferation, division, and the like. Therefore, the nucleic acid ligand according to the embodiment of the present disclosure can exhibit an effect of preventing or treating K-RAS mutant protein-expressing cancer (e.g., lung cancer, colorectal cancer, pancreatic cancer, breast cancer, ovarian cancer, endometrial cancer, cervical cancer, bladder cancer, gallbladder biliary tract cancer, skin cancer, stomach cancer, brain cancer, kidney cancer, acute myeloid leukemia, etc.).

As used herein, the term "treatment" may refer to any action that alleviates or advantageously changes symptoms of a subject with diseases.

As used herein, the term "prevention" may refer to any action that inhibits or delays the disease.

In one embodiment, the novel nucleic acid ligand may be administered at a pharmaceutically effective amount.

In one embodiment, the novel nucleic acid ligand may be administered at a dose of about 1 µg to about 100 µg, about 5 µg to about 80 µg, about 10 µg to about 70 µg, about 15 µg to about 60 µg, about 20 µg to about 50 µg, or about 30 µg to about 40 µg, and the administration may be performed once a day or divided into several times.

As used herein, the term "administration" refers to the introduction of the pharmaceutical composition according to one embodiment to a subject by any suitable method. The route of administration may be oral or parenteral through any general route as long as the pharmaceutical composition can reach a target tissue. Specifically, the pharmaceutical composition according to an embodiment may be administered orally, rectally, sublingually, and parenterally via any route. The parenteral administration may be exemplified by intravenous, intramuscular, intraarterial, intraperitoneal, intranasal, intravaginal, intravesical, intradermal, transdermal, topical, or subcutaneous administrations. Additionally, within the scope of the present disclosure, it is contemplated to administer the drug to the subject's body as a controlled formulation where systemic or localized release of the drug occurs later. For example, the pharmaceutical composition can be localized for controlled release into the circulatory system or for release to the local site of tumor growth.

In one embodiment, the pharmaceutical composition may be administered intranasally, intratracheally, or intrabronchially by inhalation. The administration by inhalation may be an administration using a pharmaceutical preparation that contains respirable particles or droplets containing the nucleic acid ligand and can be inhaled through the respiratory tract, nasal passages, or the like, but is not limited thereto. For example, a dry powder inhaler device (DPI), a pressurized metered dose inhaler (pMDI), or the like may be used for the administration by inhalation, but is not limited thereto.

As used herein, the term "pharmaceutically effective amount" refers to an amount sufficient to treat a disease at a reasonable benefit/risk ratio applicable to medical treatment, and the level of the effective dose may be determined depending on factors including the patient's body weight, sex, age, health condition, severity, or indication, activity of a drug, sensitivity to a drug, time of administration, route of administration, excretion rate, duration of treatment, and drugs that are simultaneously used, and other factors well known in the medical field.

In an embodiment, the present disclosure relates to a method of preventing or treating cancer, the method including a step of administering the novel nucleic acid ligand according to the embodiment in a pharmaceutically effective amount to a subject in need thereof.

Additionally, an embodiment of the present disclosure may relate to a nucleic acid ligand that binds to human serum albumin (HSA) and the K-RAS G12D mutant. The nucleic acid ligand may be as previously described.

In an embodiment, the nucleic acid ligand may include or have one of the nucleic acid sequences of SEQ ID NOS: 8 to 11. In an embodiment, the present disclosure relates to a use of the novel nucleic acid ligand according to an embodiment for manufacturing a medicament for preventing or treating cancer.

In an embodiment, the present disclosure relates to a novel nucleic acid ligand according to an embodiment for use in preventing or treating cancer.

An embodiment of the present disclosure pertains to a composition for cancer diagnosis, the composition containing the novel nucleic acid ligand according to one embodiment.

An embodiment of the present disclosure pertains to a kit for cancer diagnosis, the kit containing the novel nucleic acid ligand according to one embodiment.

An embodiment of the present disclosure pertains to a method for diagnosing cancer, the method including a step of administering the novel nucleic acid ligand according to one embodiment to a subject in need thereof.

An embodiment of the present disclosure pertains to a use of the novel nucleic acid ligand according to one embodiment in the manufacture of a medicine for diagnosing triple-negative cancer.

An embodiment of the present disclosure pertains to a novel nucleic acid ligand according to one embodiment for use in the diagnosis of triple-negative cancer.

An embodiment of the present disclosure pertains to a diagnostic composition containing the novel nucleic acid ligand according to one embodiment.

In one embodiment of the present disclosure, the novel nucleic acid ligand according to one embodiment can be used in the detection of diagnostic reagents, analytical reagents, harmful substances, and the like. The present disclosure in one embodiment may relate a method for detecting a target in a sample by using the novel nucleic acid ligand according to one embodiment, the method including (a) contacting the target from the sample with the novel nucleic acid ligand according to the embodiment. In one embodiment, the detection method may further include, after step (a), (b) determining whether the binding of the target and the nucleic acid ligand is formed, to detect the presence or absence of the target in the sample.

An embodiment of the present disclosure pertains to a contrast medium or diagnostic radiopharmaceutical containing a novel nucleic acid ligand according to one example. The novel nucleic acid ligand may bind to a substance that is commonly used for acting as a contrast medium or a diagnostic radiopharmaceutical, and for example, may bind to a radioactive isotope.

The pharmaceutical composition, use, and treatment methods described herein can be applied interchangeably as long as they are not contradictory.

The present disclosure will be clearly understood from the above-described embodiments and the below-described examples. Hereinafter, the present disclosure will be explained in detail such that a person skilled in the art can easily understand and implement the disclosure by way of the examples described in reference to accompanying tables. However, these examples are given for illustrating the present disclosure, and the scope of the present disclosure is not limited to these examples.

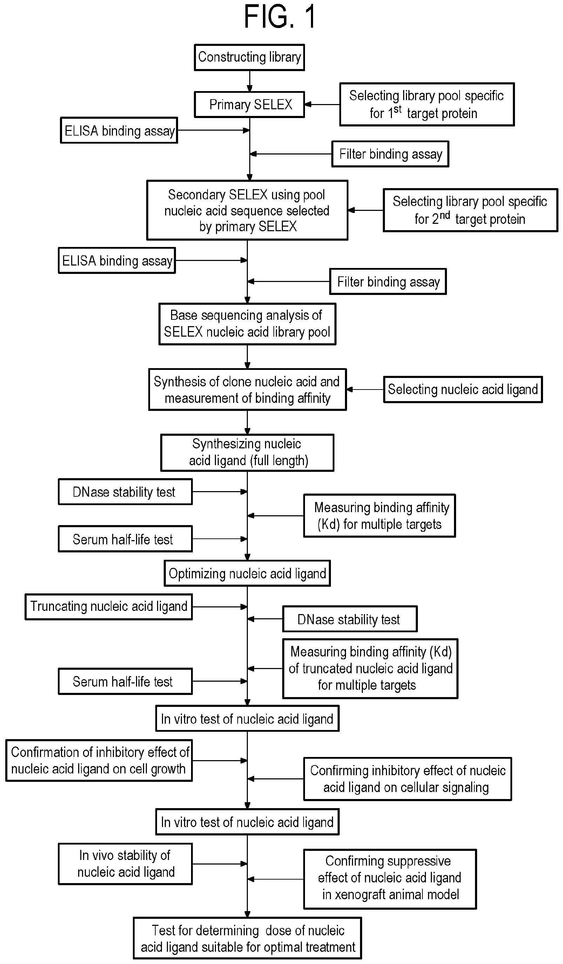

As illustrated in FIG. 1, the following working examples were carried out to obtain nucleic acid ligands according to an embodiment of the present disclosure.

### EXAMPLE 1. Selection of Novel Nucleic Acid Ligands

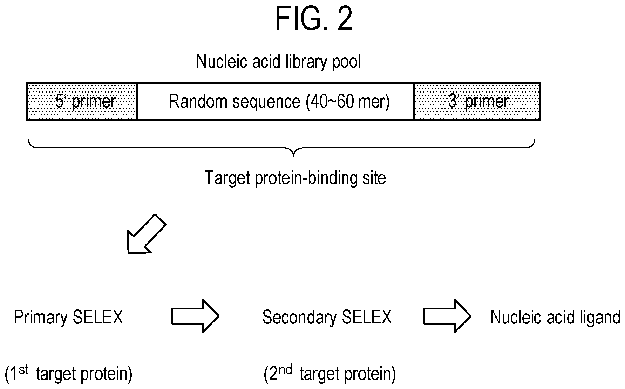

A nucleic acid library pool having binding affinity for a first target was selected from a nucleic acid library pool by using the SELEX technology (primary SELEX), and then secondary SELEX for a second target was immediately performed using the selected nucleic acid library pool, thereby ultimately obtaining nucleic acid ligands capable of binding to a plurality of targets (see FIG. 2). The selection method of novel nucleic acid ligands was specifically described below.

### 1. Construction of Modified Nucleic Acid-Containing Libraries for SELEX

A single-stranded nucleic acid library template (Biotin-GCTGGTGGTGTGGCTG-N(40)-CAGGCAGACGGTCACTCA (SEQ ID NO: 12)) was prepared with any sequence of 40 consecutive nucleotides at the center, a primer binding site for nucleic acid amplification at each of the 5'-end and 3'-end of the any sequence, and biotin conjugated in front of the primer binding site of the 5'-end. The prepared nucleic acid library template is theoretically a mixture of 1×1024 DNA with different nucleotide sequences. This single-stranded nucleic acid library template was synthesized using a DNA auto-synthesizer and used in the test after PAGE purification (Trilink, USA).

To construct a nucleic acid library containing the modified nucleic acid 5-(N-benzylcarboxamide)-2'-deoxyuridine (Bz-dU) or 5-[N-(1-naphthylmethyl)carboxamide]-2'-deoxyuridine (Nap-dU), the biotin-conjugated library template was fixed to a resin that was surface-modified with the streptavidin protein. Specifically, for the fixation, the library template and 500 µL of a resin (Thermo Scientific, USA) were mixed in a 5 M NaCl solution and reacted at room temperature for 1 hour. In addition, 20 µM 5' primer (GAGTGACCGTCTGCCTG (SEQ ID NO: 13)), 0.5 mM dNTP (dATP, dGTP, dCTP, Nap-dUTP or Bz-dUTP), 0.25 U/µL KOD XL DNA polymerase (Merck, USA), 120 mM Tris-HCl pH 7.8, and 1×KOD buffer (Merck, USA) were added to the resin washed with NaCl, followed by an enzymatic reaction at 70°C for 2 hours, thereby preparing double-stranded DNA containing modified nucleic acids. The double-stranded DNA containing modified nucleic acids was denatured by 20 mM NaOH to separate the resin and single-stranded DNA, and then the separate supernatant was neutralized with a neutralization buffer (700 mM HCl, 180 mM HEPES, 0.45% (v/v) Tween 20). The libraries that had been separated were concentrated using Amicon ultra-15 (Merck Millipore, USA) and then quantified with a UV spectrophotometer, thereby finally constructing modified nucleic acid-containing single-stranded libraries.

### 2. Selection of Nucleic Acid Library Pool Specific for First Target Protein (Plasma Protein)

### 2-1. Selection of Nucleic Acid Library Pools Specific for Plasma Proteins (Primary SELEX)

As a target protein for SELEX, a protein containing a histidine tag (6×His-tag) was used among recombinant proteins. The target protein was immobilized on beads by using Dynabeads (Thermo Scientific, USA), which were magnetic bead that were surface-modified with cobalt and binds to the His-tag of the protein. Specifically, the target protein dissolved in buffer SB18 (40 mM HEPES, 102 mM NaCl, 5 mM KC1, 5 mM MgCl2, and 0.05% (v/v) Tween 20; the same hereinafter) and 50 µl of his-tag magnetic beads were reacted at 25°C in a thermomixer (Eppendorf, Germany) at 1200 rpm for 30 minutes and washed three times with buffer SB 18 using a magnet, thereby preparing a first target protein immobilized on the beads. The first target protein is human serum albumin (HSA; Abcam, UK, product name: Recombinant human serum albumin Protein (His tag), catalog number: ab217817), which is a plasma protein. All of the above plasma proteins were used in the examples below.

The synthesized nucleic acid library pool was placed in buffer SB 18, followed by reaction from 95°C to 37°C, gradually lowered by 1°C, for 10 seconds at each temperature, thereby inducing the tertiary structure of the nucleic acid libraries. For non-specific negative selection, a protein competition buffer (1 µM prothrombin and 1 µM casein) was mixed with the above reaction solution, and then added to 10 µg of magnetic beads, followed by reaction at 37°C using a thermomixer at 1200 rpm for 10 minutes. Thereafter, the supernatant was transferred to a plasma protein-immobilized Dynabeads to select a nucleic acid library pool binding to the plasma protein. Specifically, the modified nucleic acid libraries that had been subjected to non-specific negative selection were reacted with the plasma protein-immobilized magnetic beads at 37°C for 1 hour in a thermomixer at 1200 rpm. The nucleic acid libraries and the target protein-magnetic bead complex were washed five times with buffer SB 18, and then a nucleic acid library pool binding to the target protein was collected by addition of a 2 mM NaOH solution, and then neutralized with an 8 mM HCl solution.

The libraries binding to the plasma protein were amplified using quantitative PCR (QPCR, CFX real time PCR detection system; Bio-Rad, USA) for use in the next round of SELEX. The nucleic acid libraries collected from the above SELEX process was mixed with QPCR buffer (5' primer (GAGTGACCGTCTGCCTG (SEQ ID NO: 13)), 3' primer (Biotin-GCTGGTGGTGTGGCTG (SEQ ID NO: 14)), 1xKOD buffer, KOD XL DNA polymerase, 1 and mM dNTP), followed by one cycle under the conditions of 96°C for 15 seconds, 55°C for 10 seconds, and 68°C for 30 minutes and 30 cycles repeated under the conditions of 96°C for 15 seconds and 72°C for 1 minute, thereby amplifying nucleic acid libraries binding to plasma proteins to prepare double-stranded oligo DNA.

The double-stranded oligo DNA prepared through QPCR was immobilized by mixing with 25 µl of Myone^{™} streptavidin magnetic beads (Thermo Scientific, USA; the same hereinafter) at room temperature for 10 minutes, and anti-sense strands were removed by addition of a 20 mM NaOH solution. Thereafter, a nucleic acid library pool containing modified nucleic acids was synthesized using an enzyme by the same method as in the construction of the modified nucleic acid-containing libraries, and then used in the next round. The SELEX round was repeatedly performed a total of eight times.

In the 4th to 8th SELEX rounds, a kinetic challenge for reaction with 10 mM dextran sulfate was performed after the binding of the nucleic acid library pool and the target proteins, and subsequent processes were the same as the qPCR amplification process and the anti-sense collection process in the first round.

### 2-2. Determination of Binding Affinities of Primary SELEX Nucleic Acid Pools for Plasma Proteins

### 2-2-1. Bead-Based ELISA Binding Assay: Albumin

To determine the binding affinity of the nucleic acid library pool subjected to the SELEX rounds for plasma proteins, bead-based ELISA binding assay was performed.

The nucleic acid library pools in the 5th to 7th rounds performed in advance were amplified. The nucleic acid library pool in each round was mixed with a buffer ((5' primer (GAGTGACCGTCTGCCTG (SEQ ID NO: 13), 3' primer (Biotin-GCTGGTGGTGTGGCTG (SEQ ID NO: 14)), 1XKOD buffer, KOD XL DNA polymerase, and 0.2 mM dNTP), followed by 20 cycles repeated under the conditions of 96°C for 15 seconds and 72°C for 1 minute, thereby amplifying the nucleic acid library pool to prepare double-stranded oligo DNA. The double-stranded oligo DNA was immobilized by mixing with 25 µl of Myone^{™} streptavidin magnetic beads at room temperature for 10 minutes, and sense strands in the solution were removed by addition of a 20 mM NaOH solution. Thereafter, the magnetic bead-anti-sense complex was washed two times with buffer SB 17 (40 mM HEPES, 102 mM NaCl, 5 mM KCl, 5 mM MgCl2, 1 mM EDTA, and 0.05% (v/v) Tween20; the same hereinafter), and further washed with 16 mM NaCl. The magnetic bead-anti-sense complex that had been washed was mixed with 2.5 µM 5' primer (Biotin-GAGTGACCGTCTGCCTG (SEQ ID NO: 15)), 0.5 mM dNTP (dATP, dGTP, dCTP, Nap-dUTP or Bz-dUTP), 0.25 U/µL KOD XL DNA polymerase, 120 mM Tris-HCl pH7.8, and 1×KOD buffer, followed by an enzymatic reaction at 68°C for 1 hour, thereby preparing a nucleic acid library pool containing modified nucleic acids and biotin. The supernatant was removed using a magnet and washed three times with buffer SB 17. To collect newly synthesized biotin-anti-sense strands, 20 mM NaOH was added, followed by neutralization with 80 mM HCl.

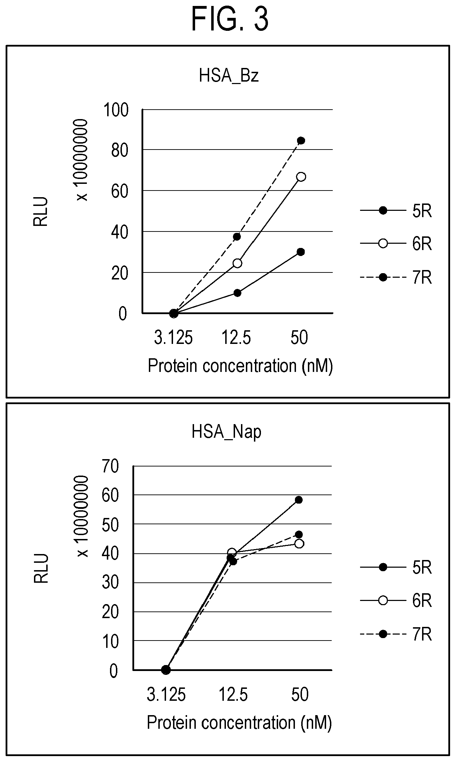

One pmol of the prepared biotin-conjugated nucleic acid library pool was placed in buffer SB18 and reacted from 95°C to 37°C, gradually lowered by 1°C, for 10 seconds at each temperature, thereby inducing the tertiary structure of DNA. The plasma proteins (HAS) were prepared with 200 nM to 4-fold dilutions, which were then bound to a structure-stabilized nucleic acid library pool at 37°C for 30 minutes, and then 10 µl of histidine pull-down beads (Thermo Scientific, USA) were placed, followed by binding at 25°C for 30 minutes. The biotin nucleic acid library pool-plasma protein-bead complex was washed three times with buffer SB 18 by using a magnet, and then 100 µl of a streptavidin-horseradish peroxidase (HRP) reagent diluted at a ratio of 1 :3,000 was added to the sample, followed by reaction at 25°C for 30 minutes. Again, the HRP-nucleic acid library pool-plasma protein-bead complex was washed three times with buffer SB 18, and then 100 µl of a chemiluminescence solution was added to separate only the supernatant by using a magnet. The supernatant was transferred to a white plate, and the fluorescence value was measured using the luminescence protocol of the GloMax plate meter (Promega, USA). Thereafter, the results are analyzed after the subtraction of a resulting value in a negative control having no protein from a resulting value for each test group (see FIG. 3).

FIG. 3 shows the target binding affinity results of the nucleic acid library pools (5R, 6R, and 7R stand for 5th, 6th, and 7th round nucleic acid library pools) containing Bz-dU (top panel) and Nap-dU (bottom panel), respectively, for the first target protein albumin. FIG. 3 confirmed the presence of nucleic acid library pools binding to target proteins in all of the primary SELEX steps, regardless of the type of modified nucleic acid or target protein used in the construction of libraries.

### 2-2-2. Filter Binding Assay: Human Serum Albumin

Filter binding assay was performed as another method for determining the binding affinity of the nucleic acid library pool subjected to the SELEX rounds for the plasma protein.

The nucleic acid library pools in the 6th to 8th rounds performed in advance were subjected to end labeling with α-32P ATP (Perkin Elmer, USA) and terminal deoxynucleotidyl transferase (TdT, New England Biolabs, USA).

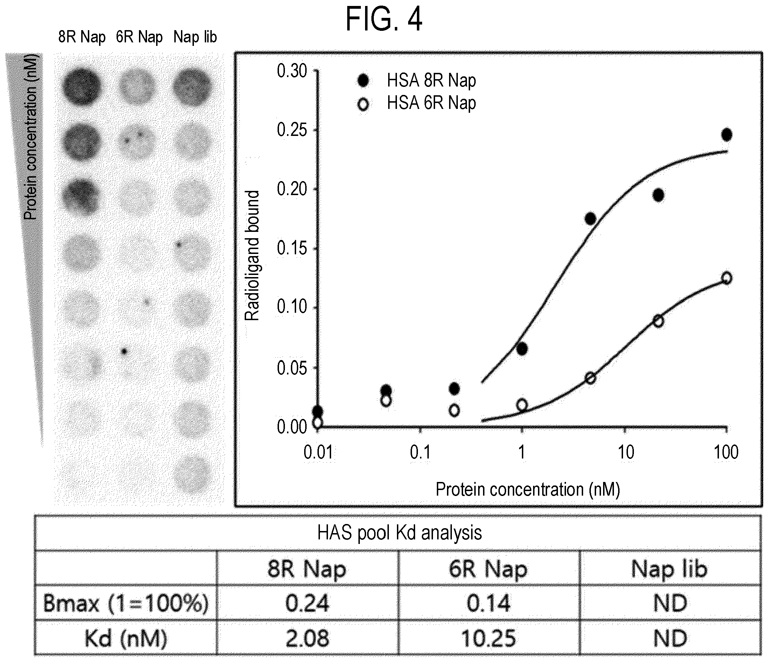

Specifically, the nucleic acid library pool obtained through the SELEX process was amplified by the same method as in 2-2-1 above, and then a sense single-stranded nucleic acid library pool was prepared. In addition, 1 pmol of the nucleic acid library pool, 0.25 µL of α-32P ATP, 0.25 µL of TdT, and NEBuffer^{™} 4 (New England Biolabs, USA) were mixed and reacted at 37°C for 30 minutes, and then the TdT enzyme was inactivated by treatment at 70°C for 10 minutes. The labeled nucleic acid library pool was purified using a Micro spin G50 column (GE Healthcare, USA) and then quantified using a beta counter instrument. The nucleic acid library pool (20,000 cpm) was placed in 100 µL of buffer SB 18 and reacted from 95°C to 37°C, gradually lowered by 1°C, for 10 seconds at each temperature, thereby inducing the tertiary structure of the nucleic acid library pool. The target protein was subjected to serial dilution with buffer SB18, and then 30 µL of the structure-stabilized labeled nucleic acid library pool was added, followed by reaction at 37°C for 30 minutes. To the mixtures of the nucleic acid library pool and the target protein was added 5.5 µL of Zorbax resin (Agilent, USA), followed by reaction for 5 minutes. The samples that had been reacted was transferred to a MultiScreen-HV Filter Plate (Merck Millipore, USA) previously wetted with 50 µL of buffer SB 18, and then the solution was removed by vacuum, and the membrane filter was washed with 100 µL of buffer SB18. The filter plate was exposed to an image plate for 16 hours, and then the image was quantified with FLA-5100 (Fujifilm, Japan). The binding affinity was calculated from the values obtained through the filter binding assay using SigmaPlot 11 (Systat Software, USA). BMax represents the ratio of bound oligo DNA to input; Kd (dissociation constant) represents affinity; and Nap lib indicates a nucleic acid library pool that has not been subjected to SELEX and was used as a negative control (see FIG. 4).

In addition, SELEX for selecting a nucleic acid library pool specifically binding to HSA, another first target protein, was performed, and then the target binding affinities of the nucleic acid library pools in the 6th and 8th rounds were determined by the above filter binding assay, and are shown in FIG. 4. FIG. 4 confirmed the presence of nucleic acid library pools showing binding affinity for the albumin protein in both of the two rounds. However, the nucleic acid library pool not subjected to SELEX showed no binding affinity for the albumin protein.

### 3. Selection of Nucleic Acid Library Pools Specific to Second Target Proteins

### (Therapeutic Target Proteins)

Secondary SELEX was performed on therapeutic target proteins by using nucleic acid library pools in rounds in which the binding affinities for plasma proteins were confirmed through the above-described primary SELEX and binding assay. The secondary SELEX method is the same as the first SELEX method, but includes a counter selection process added, if necessary, to increase binding specificity to therapeutic target proteins.

The therapeutic target protein used in the following examples was K-RAS G12D protein (Signal chem, trade name: K-RAS (G12D) Protein(R06-32BH), catalog No.: R06-32BH).

To perform secondary SELEX, the nucleic acid library pool in a specific round, which had been confirmed to have binding affinity, was needed at a sufficient amount and thus was amplified using qPCR. The process after qPCR was the same as in primary SELEX. To select a nucleic acid library pool binding to a therapeutic target KRAS mutant protein, counter selection was performed on the wild-type K-RAS protein. Specifically, the wild-type K-RAS protein was added to 20 µg of magnetic beads and reacted at 37°C by using a thermomixer at 1200 rpm for 10 minutes, and then the wild-type protein-magnetic bead complex was washed three times using a magnet and buffer SB18. To the complex thus prepared were added a mixture of the prepared nucleic acid library pool and a protein competition buffer, followed by reaction at 37°C and 1200 rpm for 10 minutes, and then a nucleic acid library pool of the supernatant, which did not bind to the wild-type protein, was transferred to the mutant target protein-immobilized beads to select a nucleic acid library pool binding to the therapeutic target protein. Secondary SELEX for the therapeutic target protein was also performed in 5 to 8 rounds by the same method, and a kinetic challenge using dextran sulfate was not performed.

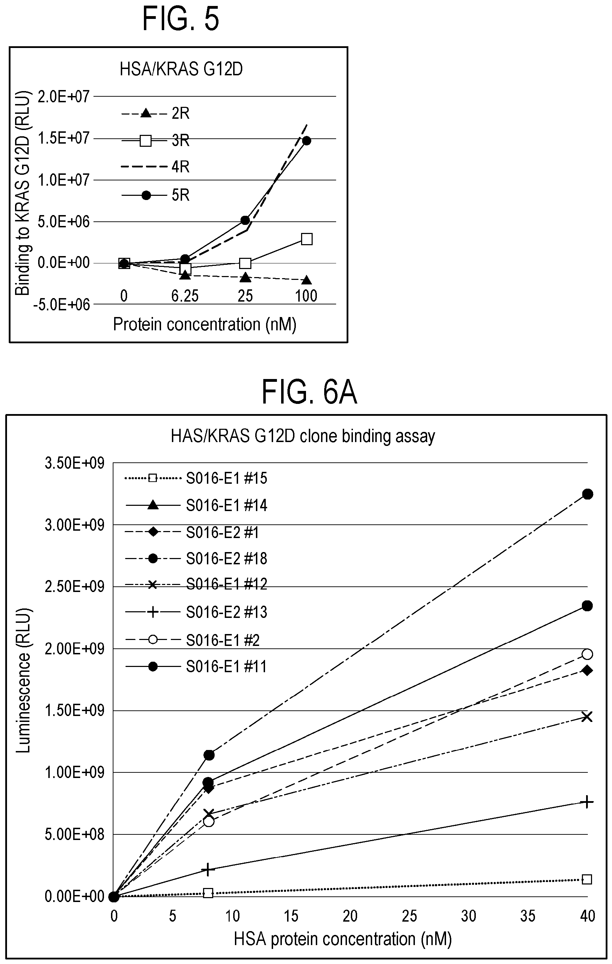

The binding affinity for therapeutic target proteins was determined using the nucleic acid library pool sample in each round by the same method as in the above-described ELISA binding assay (see FIG. 5).

For example, K-RAS G12D was used as the second target protein. The results for the second target protein are shown in FIG. 5. FIG. 5 confirmed the presence of nucleic acid library pools showing binding affinity for the second target proteins, regardless of the type of first target protein and the type of second target protein.

### 4. Nucleotide Sequencing of SELEX Nucleic Acid Library Pools

The SELEX round nucleic acid library pool samples confirmed to have binding affinity were amplified through PCR using the 5' primer (GAGTGACCGTCTGCCTG (SEQ ID NO: 13) and the 3' primer (GCTGGTGGTGTGGCTG (SEQ ID NO: 16)), cloned using the TOPO TA Cloning kit (Thermo Scientific, USA), and transformed into E. coli TOP10 cells. The plasmid nucleic acid of a single colony grown on an agar medium was amplified using a rolling circle amplification (RCA) kit (Enzynomics, Korea), and it was determined whether the cloned PCR product was inserted. Thereafter, nucleotide sequencing was performed on the RCA sample of the single sequence-inserted clones by the BigDye terminator cycle sequencing kit (Thermo Scientific, USA) and capillary electrophoresis (SolGent, Korea). For example, at least about 50 clones for each sample were sequenced to ultimately identify each nucleic acid sequence present in the nucleic acid library pools selected through SELEX.

The nucleic acid sequences that have been analyzed were analyzed by the Multalin website or our software to select sequence with the highest expression frequency (e.g., about 5 to about 10 sequences).

### 5. Synthesis of Clone Nucleic Acid Ligands Through DNA Polymerase and Determination of Binding Affinity

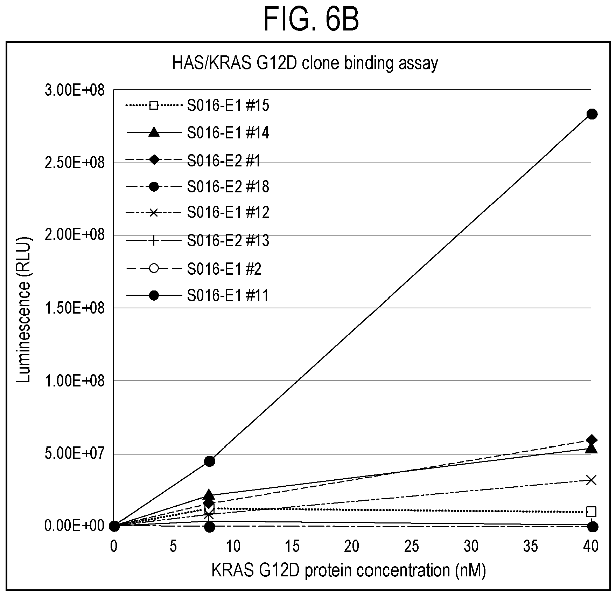

The selected nucleic acid sequences were synthesized by using a double-stranded DNA used for nucleotide sequencing as a template, along with a 5' primer (GAGTGACCGTCTGCCTG (SEQ ID NO: 13)), a biotin-conjugated primer 3' primer (Biotin-GCTGGTGGTGTGGCTG (SEQ ID NO: 14)), and DNA polymerase, and corresponded to clone nucleic acid ligands. The binding affinities of the clone nucleic acid ligands for target proteins were determined by the same method as the bead-based ELISA binding assay as in 2-2-1 of Example 1 described above (e.g., see FIGS.6A and 6B).

That is, as a result of analyzing the binding affinities of the clone nucleic acid ligands to the HSA protein and the K-RAS protein, selection was made of S016-E1 #1 clone that demonstrated the binding affinity. This clone was used to synthesize the nucleic acid ligand of SEQ ID NO: 1, which was then used in the examples below (see FIGS. 6A and 6B).

The sequences observed to have binding affinities for first and second target proteins were selected, and the full-length nucleic acid ligands thus selected were synthesized in the form of containing modified nucleic acids by using an DNA auto-synthesizer, and used as full-length nucleic acid ligands in the following examples.

For example, the full-length nucleic acid ligand selected by the method as in Example 1 was AD003, which is the nucleic acid ligand shown in Table 1.

The full-length nucleic acid ligand AD003 selected by the method of Example 1 was analyzed and purified by a reverse-phase C18 column on Waters Prep150 (Waters, USA) and Waters ACQUITY UPLC H-Class Bio PLUS System (Waters, USA). In addition, the mass spectrometry of the purified nucleic acid ligands was performed by Waters Xevo G2-XS Q-TOF System (Waters, USA). The mass spectrometry results are shown in Table 3.

In the nucleic acid sequence shown in the table below, the portion marked as "6" indicates 5-(N-(1-naphthylmethyl)carboxamide)-2'-deoxyuridine (Nap-dU).

**TABLE 2**

| Nucleic acid ligand code | 1st Target | 2nd Target | SEQ ID NO: | Nucleic acid sequence |
|---|---|---|---|---|
| AD003 | HSA | K-RAS G12D | SEQ ID NO: 1 | GAGTGACCGTC6GCCTGGAC6AGCA6AAG6CCGGA6 GGCAGC666A6GCCGACCA6CAGCCACACCACCAGC C |

### [EXAMPLE 2] Optimization (Truncation) and Preparation of Full-Length Nucleic Acid Ligands

A truncation process for optimization was performed using the full-length nucleic acid ligands selected by the methods as in Example 1.

For optimization, the 2D structures of the full-length nucleic acid ligands were predicted using the Mfold web server (http://www.unafold.org/mfold/applicationsidna-folding-form.php), and the length was reduced from both ends of the full-length nucleic acid ligands selected on the basis of the structures, to synthesize optimized nucleic acid ligand candidates with minimum lengths similar to the clone sequence (the full-length nucleic acid ligand serving as the parent). For example, the 2D prediction structures of the nucleic acid ligands AD003 to AD008 are shown in FIGS. 7A to 7C.

The nucleic acid ligands were synthesized by a general DNA auto-synthesis method using Mermade 12 or Mermade 48 (Bioautomation, USA). The nucleic acid ligand was sequentially synthesized from the 3'-end to the 5'-end by four steps (deblocking->coupling->capping->oxidation) as a single cycle for each 1-mer (each one nucleotide). If necessary, biotin or a fluorescent substance (FAM or Cy5) was conjugated in front of the 5'-end of the nucleic acid ligand. The biotin and FAM were synthesized in an auto-synthesizer, and Cy5 was synthesized by amine coupling after 5'-amine nucleic acid ligand synthesis. The synthesized nucleic acid ligand or the biotin or fluorescent substance (FAM or Cy5)-conjugated nucleic acid ligand was purified by HPLC and used in the following example.

The nucleic acid ligands prepared in Example 2, together with full-length nucleic acid ligand AD003 (indicated in bold and underlined), are shown in Table 3. All of optimized nucleic acid ligands in Table 3 used the same full-length nucleic acid ligand AD003.

The nucleic acid ligands thus prepared were analyzed and purified by a reverse-phase C18 column on Waters Prep150 (Waters, USA) and Waters ACQUITY UPLC H-Class Bio PLUS System (Waters, USA). In addition, the mass spectrometry of the purified nucleic acid ligands was performed by Waters Xevo G2-XS Q-TOF System (Waters, USA). The mass spectrometry results are shown in Table 3.

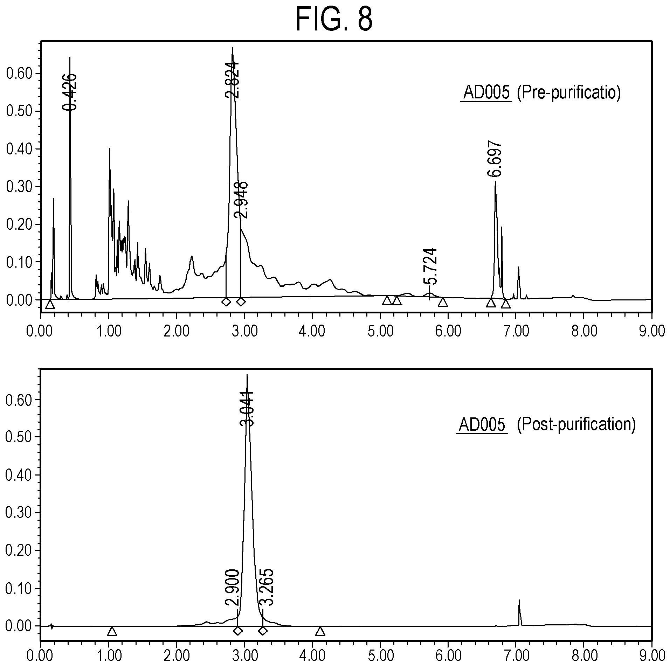

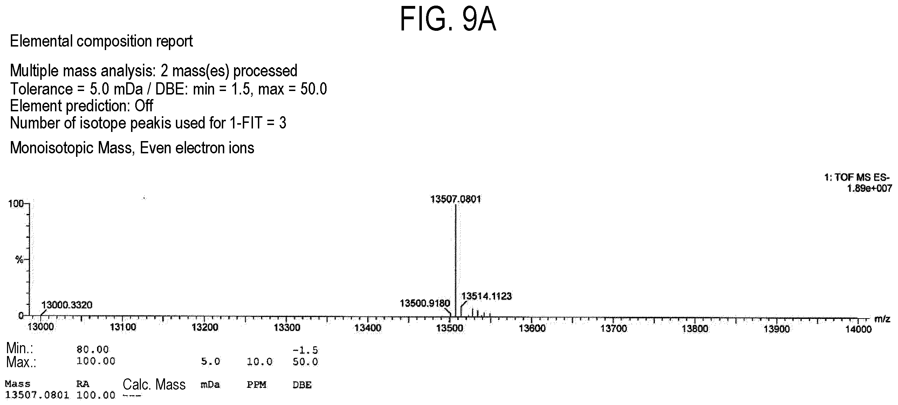

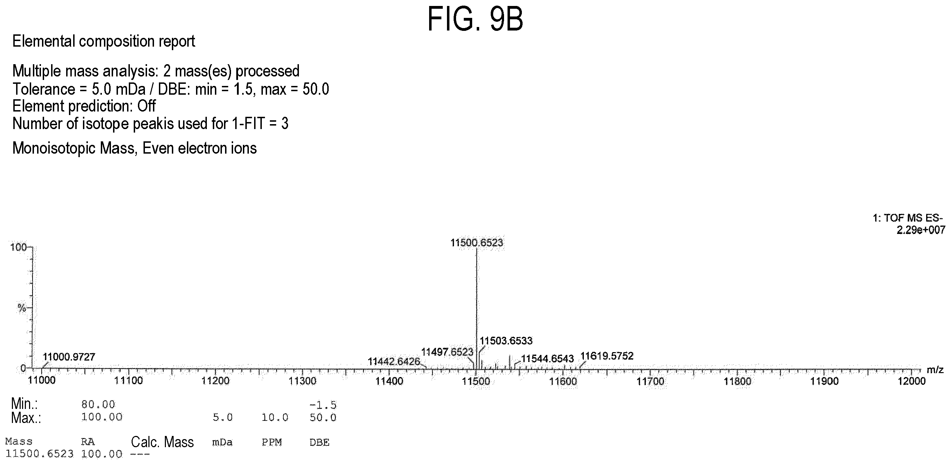

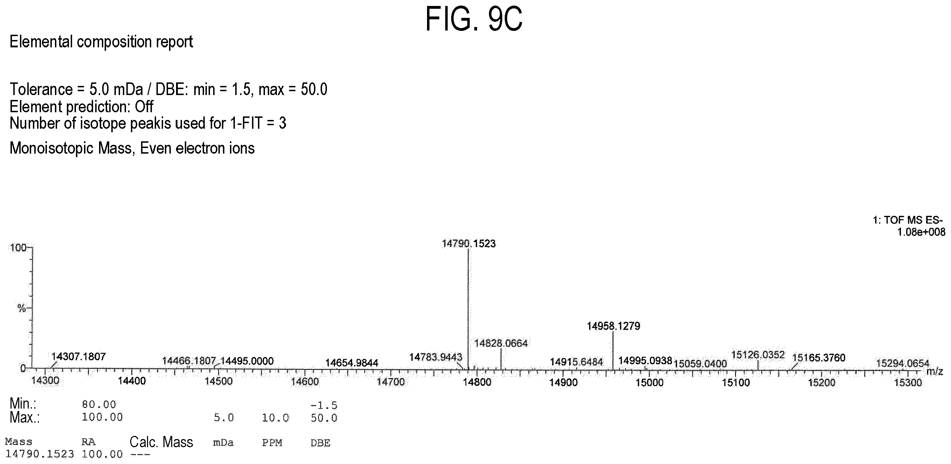

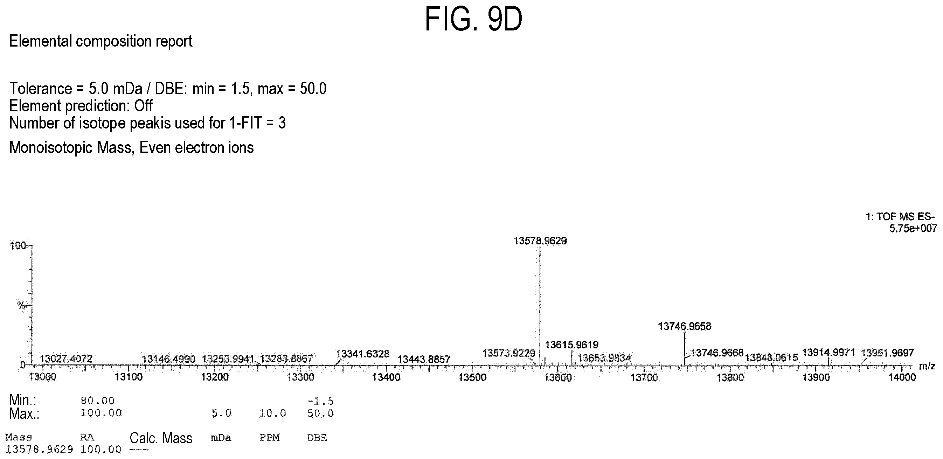

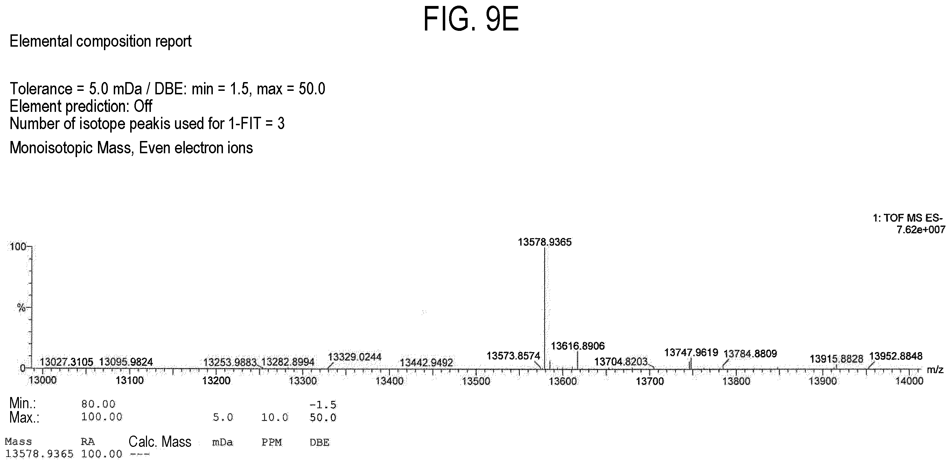

For example, the UPLC analysis results, before and after purification, of the nucleic acid ligand AD005 according to one embodiment of the present disclosure are shown in FIG. 8 and the mass spectrometry results thereof are shown in FIGS. 9A to 9E.

**TABLE 3**

| Nucleic acid ligand code | 1st Target | 2nd Target | SEQ ID NO: | Nucleic acid sequence | Mw. Calculated (Da) | Mw. Measured (Da) |
|---|---|---|---|---|---|---|
| AD003 | HSA | K-RAS G12D | SEQ ID NO: 1 | | 24457.40326 | 24457.0605 |
| AD004 | HSA | K-RAS G12D | SEQ ID NO: 2 | | 16962.14036 | 16962.0449 |
| AD005 | HSA | K-RAS G12D | SEQ ID NO: 3 | | 13912.64727 | 13912.1592 |
| AD006 | HSA | K-RAS G12D | SEQ ID NO: 4 | | 12235.3405 | 12234.8760 |
| AD007 | HSA | K-RAS G12D | SEQ ID NO: 5 | | 11409.17794 | 11408.7393 |
| AD008 | HSA | K-RAS G12D | SEQ ID NO: 6 | | 9899.928681 | 9899.9736 |
| AD089 | HSA | K-RAS G12D | SEQ ID NO: 7 | | 11506.9764 | 11500.6523 |
| AD210 | HSA | K-RAS G12D | SEQ ID NO: 8 | | 14798.0627 | 14790.1523 |
| AD211 | HSA | K-RAS G12D | SEQ ID NO: 9 | | 13586.2999 | 13578.9629 |
| AD212 | HSA | K-RAS G12D | SEQ ID NO: 10 | | 13586.2999 | 13578.9365 |

| | | | | | | |
|---|---|---|---|---|---|---|
| * In the mass spectrometry results of Table 3, the nucleic acid ligands having biotin conjugated to the 5' end thereof were used. Hereinafter, a procedure for selecting nucleic acid ligands capable of exhibiting efficacy when actually used as a drug was performed on the nucleic acid ligands prepared in Examples 1 to 2. | | | | | | |

### EXAMPLE 3. Assay for DNnase Stability of Selected Full-Length Nucleic Acid Ligands Against Plurality of Targets

There are several types of nucleases in serum, and it is well known that the reaction of oligonucleic acid materials with serum results in the degradation of oligonucleic acid molecules within several hours. However, it has been reported that a nucleic acid ligand conjugated to a plasma protein such as albumin is not degraded by exhibiting resistance to the degradation action of nucleases. The nucleic acid ligands, even when conjugated to target proteins other than plasma proteins, can exhibit resistance to the degradation action of nucleases. Therefore, the selected full-length nucleic acid ligands were reacted under conditions of exposure to a nuclease to checking the degradation degree of the nucleic acid ligands, thereby evaluating the presence or absence of the binding with first and second target proteins and furthermore evaluating the stability in serum. These evaluations can determine in advance whether a nucleic acid ligand can exhibit valid efficacy by binding to a target protein in the blood to maintain its structure or its target binding site, which exhibits the desired effect.

First, 0.5 pmoles of a nucleic acid ligand (selected by the method as in Example 1 and 2) dissolved in buffer SB 18, DMEM, a buffer solution for nuclease reaction (DNaseI RNase-free, Thermo scientific, USA), and sterile DW were mixed. The nucleic acid ligand mixture thus prepared was reacted from 95°C to 37°C, gradually lowered by 1°C, for 10 seconds at each temperature, thereby inducing the tertiary structure of the nucleic acid ligand. To the stabilized nucleic acid ligand was added 20 pmoles of a first target protein (a plasma protein) or a second target protein (a therapeutic target protein), followed by reaction at 4°C for 30 minutes, thereby conjugating the target protein to the nucleic acid ligand. The target protein was not added for a positive control and a negative control. To this reaction solution was added 1 unit of a nuclease (DNaseI, Thermo Scientific, USA), thereby activating the action of the nuclease at 37°C for 16 hours. The nuclease was not added for the negative control. After the 16-hour reaction, the nuclease was inactivated by heating at 85°C, and electrophoresis was performed on a 10% urea-polyacrylamide gel (Urea-Polyacrylamide gel). The stability of the nucleic acid ligand against the nuclease was determined by comparing the band intensities of the respective controls and respective test groups. From the above results, whether the nucleic acid ligand binds to the target protein with high affinity could be confirmed, and nucleic acid ligand candidates exhibiting excellent stability against the nuclease could be selected.

The above test was conducted using the full-length nucleic acid ligands selected by the methods as in Example 1. The nucleic acid ligands having significant band intensities for both the plasma protein and the therapeutic target protein would have excellent binding characteristics to both the plasma protein and therapeutic target protein. The binding to each target protein was evaluated to be significant when the band intensity was 10% or more in comparison with the band of the negative control without the addition of the nuclease, in the electrophoresis results. Typical full-length nucleic acid ligands showing binding to both the plasma protein and the therapeutic target protein are shown in Table 4. Even though the DNase electrophoresis results for the full-length nucleic acid ligands are not significant, the optimized sequences thereof might show significant electrophoresis results and confirmed binding affinity (Kd).

For example, the electrophoresis results with respect to DNase stability for AD003 to AD008 nucleic acid ligands are shown in FIGS. 7A to 7C.

To further determine the binding affinity for a target of the nucleic acid ligands showing binding to each target protein, the biding affinity (Kd) was measured.

### EXAMPLE 4. Binding Affinity (Kd) of Full-Length Nucleic Acid Ligands for Plurality of Targets

The binding affinity of the nucleic acid ligands were measured using enzyme linked immunosorbent assay (ELISA). First, the 5' biotin-conjugated nucleic acid ligand was reacted in buffer SB18 from 95°C to 37°C, gradually lowered by 1°C, for 10 seconds at each temperature, thereby inducing the tertiary structure of the nucleic acid ligand.

The test method was composed of the following steps.
1) A target protein was coated on a microplate at a low temperature for 16 hours.
2) The surface of the microplate was blocked by reaction at room temperature for 1 hour using Blocking Buffer (Pierce^{™} Protein-Free (PBS) Blocking Buffer, Thermo Scientific, USA).
3) The microplate was treated with a nucleic acid ligand, followed by reaction at 37°C for 1 hour.
4) For enzyme conjugation, the microplate was treated with a streptavidin-horseradish peroxidase (HRP) conjugate (Thermo Scientific, USA), followed by reaction at room temperature for 1 hour.
5) The microplate was treated with TMB substrate solution (Thermo Scientific, USA), followed by reaction at room temperature for 30 minutes.
6) The microplate was treated with a reaction termination solution (2 M sulfuric acid).
7) The absorbance was measured at 450 nm using GloMax plate meter (Promega, USA).

The target protein and the nucleic acid ligand were each used at 4-fold dilution starting from 500nM. The measured binding affinities of the nucleic acid ligands for respective target proteins are shown in Table 4. It could be confirmed that the nucleic acid ligands showing the binding to all the target proteins in the electrophoresis results exhibited binding affinity for each target protein.

### EXAMPLE 5. Serum Half-Life of Full-Length Nucleic Acid Ligands

First, 2 pmoles of the nucleic acid ligand dissolved in buffer SB 18 was reacted from 95°C to 37°C, gradually lowered by 1°C, for 10 seconds at each temperature, thereby inducing the tertiary structure of the nucleic acid ligand. The stabilized nucleic acid ligand was added to the 96% human serum and then reacted at 37°C for 0, 3, 6, 9, 12, 15, and 18 hours, thereby inducing the degradation of the nucleic acid ligand. After each of the reaction times, the nuclease was inactivated by heating at 85°C, and electrophoresis was performed on a 10% urea-polyacrylamide gel. A control without the addition of human serum was included as a loading control, and the band intensities of the control and the test groups were measured. The intensity trend line over time was determined using the measurement values, and the intensity value was inserted into the trend equation to obtain the half-life. If the intensity value deviates from the trend line, an additional test was conducted under conditions of 0, 24, 48, 72, and 96 hours.

For example, the electrophoresis results over time (results of serum stability assay) for the AD003 to AD008 nucleic acid ligands are shown in FIGS. 7A to 7C. In addition, the serum half-life (hr) thus measured for each target protein is shown in Table 4. Table 4 confirmed that the nucleic acid ligands according to one embodiment of the present disclosure showed high serum half-life, indicating that the nucleic acid ligands according to one embodiment of the present disclosure can have excellent binding affinity for the plasma proteins and thus show excellent in vivo stability.

### EXAMPLE 6. Binding Affinity (Kd), Serum Half-Life, and DNase Stability of Optimized Nucleic Acid Ligands

Some of the optimized nucleic acid ligands prepared in Example 2 were tested for DNase stability for target proteins by the same method as in Example 3. The results for typical optimized nucleic acid ligands are shown in Table 4.

The optimized nucleic acid ligands confirmed to have binding to target proteins according to the DNase stability test were measured for binding affinities (Kd) for target proteins by the same method as in Example 4 and determined for the serum half-life by the same method as in Example 5. The results thus obtained are shown in Table 4.

The nucleic acid ligands indicated in bold and underlined on Table 4 were the original full-length nucleic acid ligands (full length sequences), which were the basis for optimization, and the nucleic acid ligands therebelow corresponded to the optimized truncated nucleic acid ligands (truncated sequences).

**TABLE 4**

| Nucleic acid ligand code | 1st Target | 2nd Target | SEQ ID NO: | Serum half-life t1/2 (hr) | Biding affinity for 1 st target | | Biding affinity for 2nd target | |
|---|---|---|---|---|---|---|---|---|
| | | | | | Kd(nM) | R2 | Kd(nM) | R2 |
| AD003 | HSA | K-RAS G12D | SEQ ID NO: 1 | 49.81 | 13.01 | 91.2 | 13.79 | 91.3 |
| AD004 | HSA | K-RAS G12D | SEQ ID NO: 2 | 72 or more | 190.44 | 89.7 | 16.59 | 97.4 |
| AD005 | HSA | K-RAS G12D | SEQ ID NO: 3 | 30.1 | 121.90 | 94.9 | 22.92 | 92.8 |
| AD006 | HSA | K-RAS G12D | SEQ ID NO: 4 | 17.4 | 938.84 | 75.9 | 77.56 | 93.3 |
| AD007 | HSA | K-RAS G12D | SEQ ID NO: 5 | 12.1 | 535.83 | 97.0 | 151.68 | 98.4 |
| AD008 | HSA | K-RAS G12D | SEQ ID NO: 6 | 17.2 | 66.86 | 96.6 | 286.65 | 97.1 |

| | | | | | | | | |
|---|---|---|---|---|---|---|---|---|
| **[0215]** * In the test shown in Table 4, nucleic acid ligands having biotin conjugated to the 5'-end thereof were used. Then, of the nucleic acid ligands of SEQ ID NOS: 1 to 10 prepared in the foregoing, the optimized ligands AD005 (SEQ ID NO: 3), AD0089 (SEQ ID NO: 7), AD210 (SEQ ID NO: 8), AD211 (SEQ ID NO: 9), and AD212 (SEQ ID NO: 10) were examined for anti-cancer efficacy against triple-negative breast cancer. | | | | | | | | |

### EXAMPLE 7. Assay for Anticancer Efficacy of Human Serum Albumin (HSA)- and K-RAS G12D0-Targeting Nucleic Acid Ligand in Triple Negative Breast Cancer Orthotopic Model

The selection of in-vivo experimental models is crucial for evaluating the anticancer efficacy against triple-negative breast cancer (TNBC). Due to the complex immunological and physiological actions in the human body, the use of a model that closely resembles the human environment is necessary to reliably infer anticancer efficacy in humans from animal preclinical experimental results. The model selection is especially important particularly for TNBC, which is invasive, metastatic, and recurrent, with a high probability of early recurrence and mortality in remote organs.

Currently available preclinical spontaneously metastatic TNBC orthotopic murine models include the immunodeficient human-derived MDA-MB-231 model and the immunocompetent murine 4T1 model. The human-derived MDA-MB-231 cell line, which is a xenograft in mice, requires the use of NOD/SCID or nude mice. The xenografted cancer cells are not inherently invasive or aggressive unlike those in mice, as the host is not human. Therefore, Matrigel is typically used during xenografting because human-derived cancer cells do not readily engraft in mice. The MDA-MB-231 model does not grow as quickly in mice as mouse cells like 4T1, CT26, MC38, B16F10, etc. The relative lack of invasiveness/aggressiveness in the mouse model makes metastasis occur less frequently therein. NOD/SCID or nude mice that need to be used for xenografting do not give reliability for assay results of anticancer efficacy in humans because their immune system is deleted or do not operate normally due to the lack of some or all immune cells. This is particularly true for invasive and metastatic cancers like TNBC, as the model might not fully replicate and evaluate the cancer's occurrence and anticancer mechanisms. Additionally, since immune cells contribute to the mechanism of cancer metastasis, the MDA-MB-231 model, which necessitates the use of immunodeficient mice, has limitations in evaluating efficacy against TNBC compared to the 4T1 model and may not yield accurate evaluation results. Therefore, in this experiment, the 4T1 mouse model was used to evaluate the prophylactic or therapeutic effect of the nucleic acid ligand of the present disclosure on primary and metastatic TNBC (i.e., cancer metastasis to other organs or metastatic cancer occurring in other organs even after surgical removal). In other words, the 4T1 model is not only a primary TNBC model for targeted treatment of the primary tumor that grows after injecting 4T1 tumor cells into the mammary fat pad (4th fat pad) of mice, but also a metastatic breast cancer model, in which the 4T1 tumor cells grown as above are used. When the primary tumor is completely removed surgically, the remaining tumor cells spontaneously metastasize to other organs, especially the lungs, creating a breast cancer spontaneous metastasis model. In this regard, when 4T1 cells tagged with the luciferase reporter gene are employed, imaging can be performed at various time points using luciferin and an in vivo imaging system (IVIS). Thus, 4T1 mouse model is a useful model for observing the effects of anticancer drugs on primary tumor treatment and breast cancer metastasis treatment.

### Cell Culture

4T1-Luc2 triple-negative cell line, obtained from ATCC (USA), was used in the experiments. The cells were cultured in a medium supplemented with 10% FBS and 1% antibiotics at 37°C in a 5% CO2 incubator. When the confluency reached 80% or more, passages were performed as follows. Cells washed with PBS were treated with 0.05% trypsin-EDTA for 3 minutes, and then a fresh medium was added to inactivate the trypsin. The cells were collected using a centrifuge, diluted in a new culture vessel at a 1:5 ratio, and cultured. All cells were tested for mycoplasma contamination by PCR using a kit (Biomax, Korea), and only the cells free of contamination were used for experiments.

### Animal Preparation

Female Balb/c-nu mice (7 weeks old) were purchased from ORIENT BIO (Seongnam, Korea). All animal experiments were conducted in accordance with the regulations of the Institutional Animal Care and Use Committee (IACUC) of Sungkyunkwan University.

### Example 7-1: Assay for Anticancer Efficacy of Human Serum Albumin (HSA)- and K-RAS G12D-Targeting Nucleic Acid Ligand in Triple-Negative Breast Cancer Orthotopic Mouse Model (Non-Tumor Resection Model)

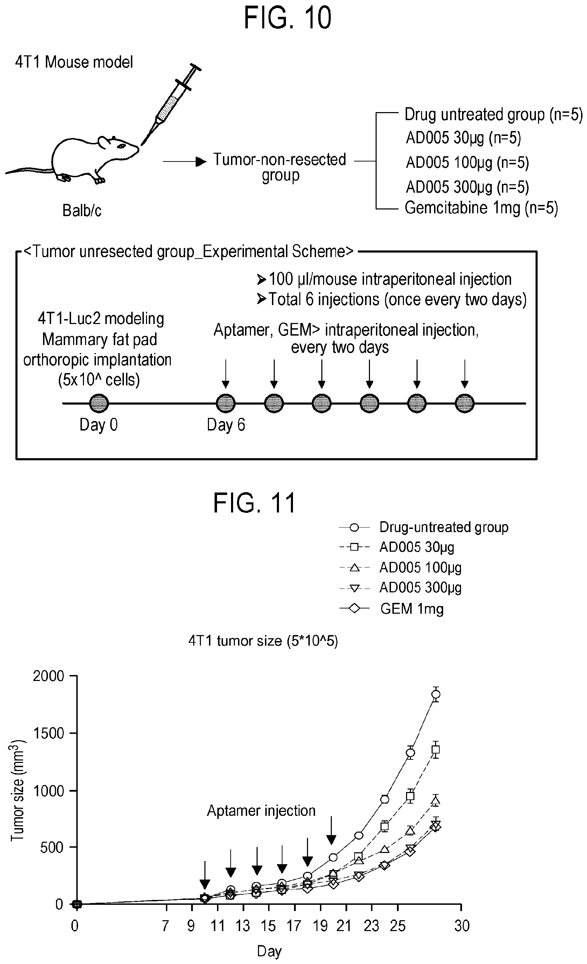

### 7-1-1. Construction of Triple-Negative Breast Cancer Orthotopic Mouse Model (Non-Tumor Resection Model) and Drug Administration thereto (see FIG. 10)

The 4T1-Luc2 cell line, cultured as described in the foregoing, was diluted in 30µl of 1X PBS (Hyclone) (5X105 cells/30µl for each mouse). These cells were orthotopically injected into the 4th fat pad of the prepared female Balb/c mice. Six days post-injection (when the average tumor size reached approximately 60mm3), the mice were intraperitoneally injected with either control drugs or the test compound to compare tumor suppression efficacy.

Each test group consisted of five animals. The positive control drug, gemcitabine (1mg), and the test compound AD005 (30*µ*g, 100*µ*g, 300*µ*g) were administered intraperitoneally every other day for a total of six injections.

### 7-1-2. Observation and Measurement of Results Post Drug Administration

The survival rate and weight changes of the mice were observed for approximately 30 days, and tumor volume was measured every two days using the equation [longest tumor axis X (shortest tumor axis)2 X 0.5].

### 7-1-3. Experimental Results

The results of the tumor suppression efficacy in the non-tumor resection model are shown in FIG. 11.

The untreated group showed a rapid increase in tumor size over time. In contrast, the group treated with the nucleic acid ligand of the present disclosure showed a significant reduction in tumor growth, with the test compound AD005 exhibiting an increasingly effective tumor suppression effect as the dosage increased. Notably, the 300*µ*g treatment group of AD005 showed a similar level of tumor suppression efficacy to the positive control group treated with gemcitabine, despite having approximately one-third of the dosage (FIG. 11).

### Example 7-2: Assay for Anticancer Efficacy of Human Serum Albumin (HSA)- and K-RAS G12D-Targeting Nucleic Acid Ligand in Triple-Negative Breast Cancer Orthotopic Mouse Model (Tumor Resection Model)

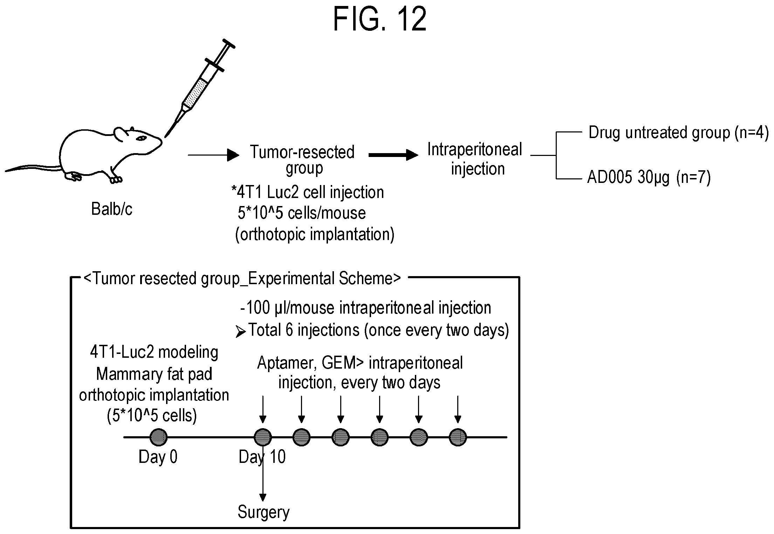

### 7-2-1. Construction of Triple-Negative Breast Cancer Orthotopic Mouse Model (Tumor Resection Model) and Drug Administration thereto (see FIG. 12)

The 4T1-Luc2 cell line, cultured as described in the foregoing, was diluted in 30µl of 1X PBS (Hyclone) (5X105 cells/30µl for each mouse) and orthotopically injected into the 4th fat pad of prepared female Balb/c mice. Ten days post-injection (when the average tumor size reached approximately 60mm3), the tumors were surgically removed, followed by intraperitoneal injection of the test compound to compare the efficacy in inhibiting tumor metastasis.

The composition of the test groups is as shown in Table 5. The test compound AD005 at 30*µ*g was administered intraperitoneally every other day for a total of six injections (Days 0, 2, 4, 6, 8, and 10). The metastasis status of each test group was monitored using IVIS (IVIS Lumina XRMS, Perkin Elmer).

**TABLE 5**

| Group | Test substance | No. of animal | Administration route | Dose (*µ*g /head) | No. of administration |
|---|---|---|---|---|---|
| G1 | Drug untreated | 4 | - | - | - |
| G2 | AD005 30*µ*g | 7 | Intraperitoneal | 30 | 6 |

### 7-2-2. Mouse Observation and Measurement of Results Post Drug Administration

Post intraperitoneal drug administration, in vivo imaging was performed using IVIS at approximately 7-day intervals until 50 days had elapsed. Mice were injected with luciferin at a concentration of 150mg/kg per mouse, and IVIS imaging was conducted 10 minutes after injection.

### 7-2-3. Experimental Results

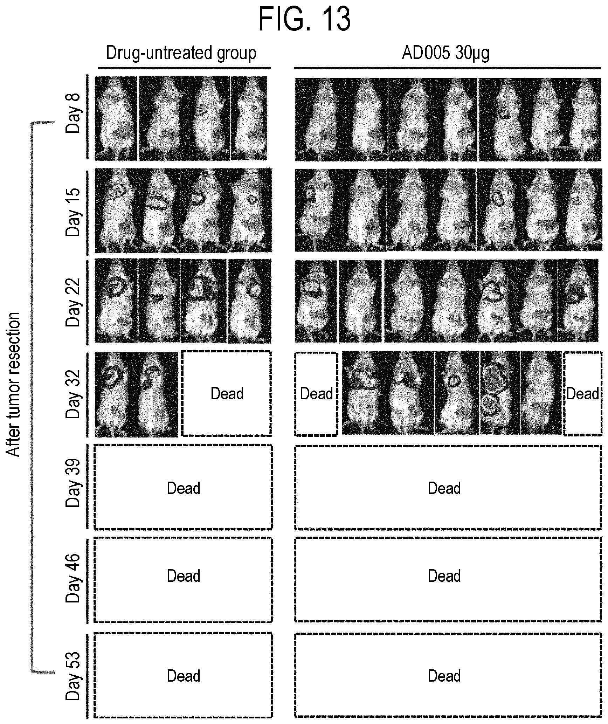

The results of the tumor resection model experiment in inhibiting metastasis and recurrence are shown in FIG. 13. The untreated group showed a 50% survival rate after 32 days, with 2 out of 4 mice deceased post-tumor resection. However, the group treated with 30µg of the test substance AD005 demonstrated a higher survival rate of approximately 70%, with only 2 out of 7 mice deceased at the same 32-day mark.

Example 7-3: Assay for Anticancer Efficacy of Human Serum Albumin (HSA)- and K-RAS G12D-Targeting Nucleic Acid Ligand and Truncated Variant thereof (AD005 Truncated sequence) in Triple-Negative Breast Cancer Orthotopic Animal Model (Tumor-Resected Model)

### 7-3-1. Construction of Triple-Negative Breast Cancer Orthotopic Animal Model (Tumor-Resected Model) and Substance Administration thereto

The method of creating the Triple-Negative Breast Cancer Orthotopic Animal Model (Non-resection Model) and the route of substance administration are as described in 7-2-1.

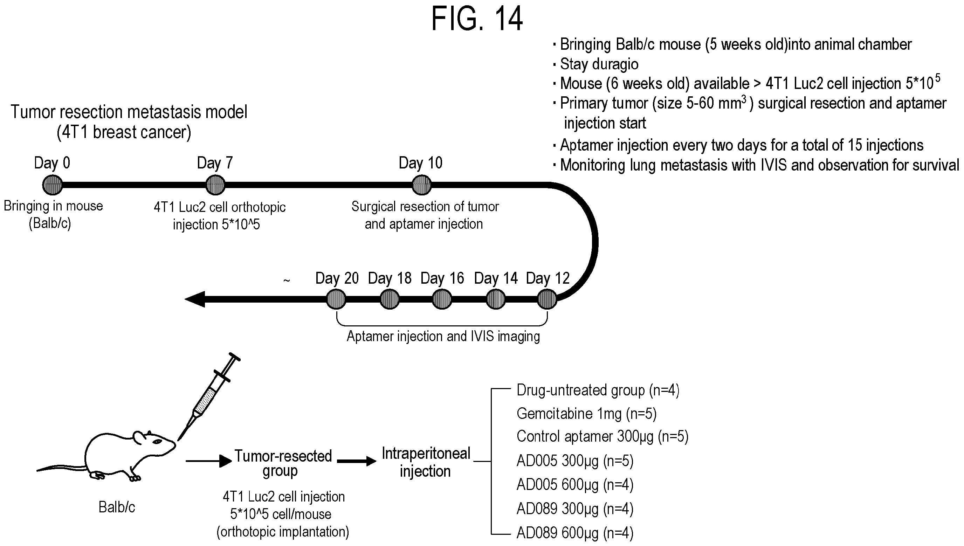

The test group compositions are as shown in Table 6. The positive control drug gemcitabine (1mg), Control aptamer (SEQ ID NO: 17) (300µg), which is a negative control drug (ERBB2 targeting nucleic acid ligand), and test substances AD005 (300µg, 600µg) and AD089 (300µg, 600µg) were injected intraperitoneally every two days for a total of 15 injections (Days 0, 2, 4, 6, 8, 10, 12, 14, 16, 18, 20, 22, 24, 26, and 28). The metastasis status of each test group was monitored using IVIS (IVIS Lumina XRMS, Perkin Elmer). The experimental plan overview is schematically shown in FIG. 14.

**TABLE 6**

| Group | Test substance | No. of animal | Administration route | Dose (*µ*g/head) | No. of administration |
|---|---|---|---|---|---|
| G1 | Drug untreated | 4 | - | - | - |
| G2 | Gemcitabine | 5 | Intraperitoneal | 1000 | 15 |
| G3 | Control aptamer | 5 | Intraperitoneal | 300 | 15 |
| G4 | AD005 300*µ*g | 5 | Intraperitoneal | 300 | 15 |
| G5 | AD005 600*µ*g | 4 | Intraperitoneal | 600 | 15 |
| G6 | AD089 300*µ*g | 4 | Intraperitoneal | 300 | 15 |
| G7 | AD089 600*µ*g | 4 | Intraperitoneal | 600 | 15 |

### 7-3-2. Mouse Observation and Result Measurement After Substance Administration

Post-intraperitoneal drug administration, the mice were imaged using IVIS (in vivo imaging system) at intervals of approximately 7 days for a total of 59 days. Each mouse was injected with luciferin at a concentration of 150mg/kg, and images were taken 10 minutes after the reaction.

The survival rate and weight changes of the mice were observed for about 60 days, and tumor volume was measured using the equation [longest diameter of the tumor X (shortest diameter of the tumor)2 X 0.5].

### 7-3-3. Experiment Results

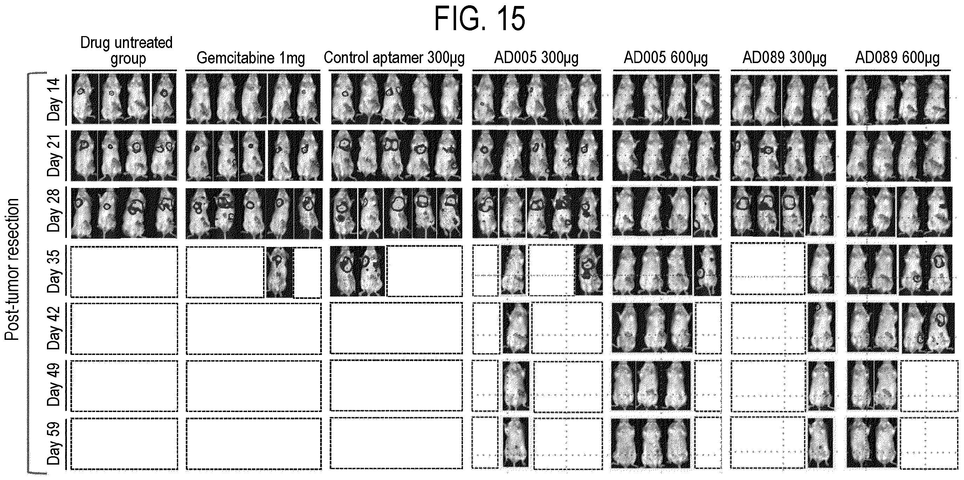

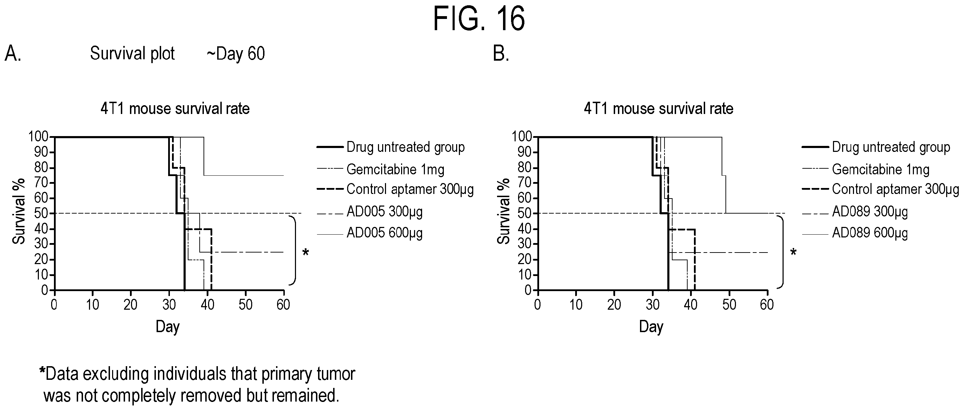

The results of metastasis and recurrence inhibition in the tumor resection model experiment are as shown in FIG. 15 and FIGS. 16A and 16B. The untreated group perished before 35 days post-tumor resection, while both the groups treated with gemcitabine, which is the positive control, and Control aptamer, which is the negative control (HER2 targeting nucleic acid ligand) perished due to metastasis before 42 days. However, some individuals in the groups treated with test substances AD005 and AD089 survived until 59 days.

As of 59 days, the AD005 300µg-administered group showed a 20% survival rate with 4 out of 5 mice deceased, and the AD005 600µg-administered group had a 75% survival rate with only 1 out of 4 mice deceased. Additionally, the AD089 300µg-administered group exhibited a 25% survival rate with 3 out of 4 mice deceased, and the AD089 600µg-administered group had a 50% survival rate with 2 out of 4 mice deceased.

When compared to the untreated group and the control groups, the test substances AD005 and AD089 demonstrated increasingly effective anti-metastasis and anti-recurrence effects with higher dosages.

### Example 7-4: Assay for Anticancer Efficacy of Novel Substance Having Gemcitabine Bound or Substituted at Any Position on Human Serum Albumin (HSA)- and K-RAS G12D-Targeting Nucleic Acid Ligands in Triple-Negative Breast Cancer Orthotopic Animal Model (Tumor Resection Model)

### 7-4-1. Construction of Triple-Negative Breast Cancer Orthotopic Animal Model (Tumor Resection Model) and Substance Administration thereto

The method of constructing the Triple-Negative Breast Cancer Orthotopic Animal Model (Tumor Resection Model) is the same as described in 7-2-1.

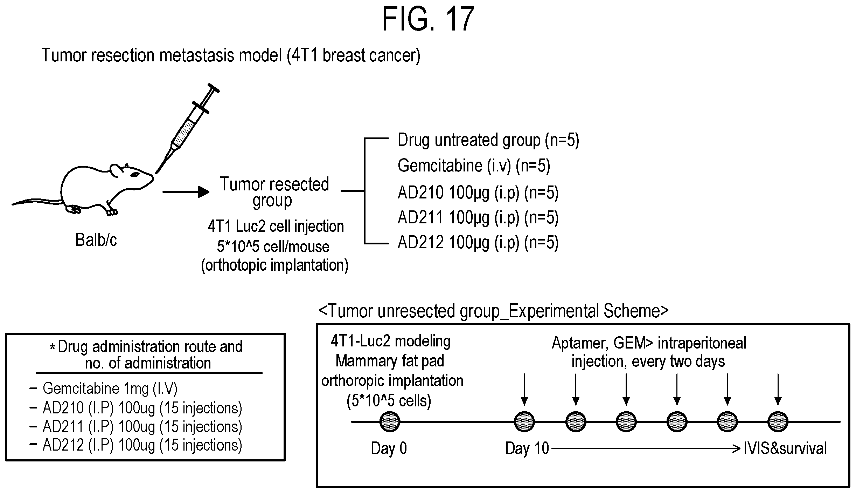

Each test group consisted of 5 animals. The positive control drug gemcitabine (1mg) was injected intravenously (IV injection) every two days for a total of 10 injections (Days 0, 2, 4, 6, 8, 10, 12, 14, 16, and 18). The test substances AD210 (100µg), AD211 (100µg), and AD212 (100µg) were injected intraperitoneally every two days for a total of 15 injections (Days 0, 2, 4, 6, 8, 10, 12, 14, 16, 18, 20, 22, 24, 26, and 28). The metastasis status of each group was monitored using IVIS (IVIS Lumina XRMS, Perkin Elmer). The experimental plan overview is illustrated in FIG. 17.

### 7-4-2. Mouse Observation and Result Measurement After Substance Administration

After intraperitoneal administration, the mice were imaged using IVIS (in vivo imaging system) at intervals of approximately 7 days for a total of 40 days. Each mouse was injected with luciferin at a concentration of 150mg/kg, and images were taken 10 minutes after the reaction.

### 7-4-3. Experiment Results

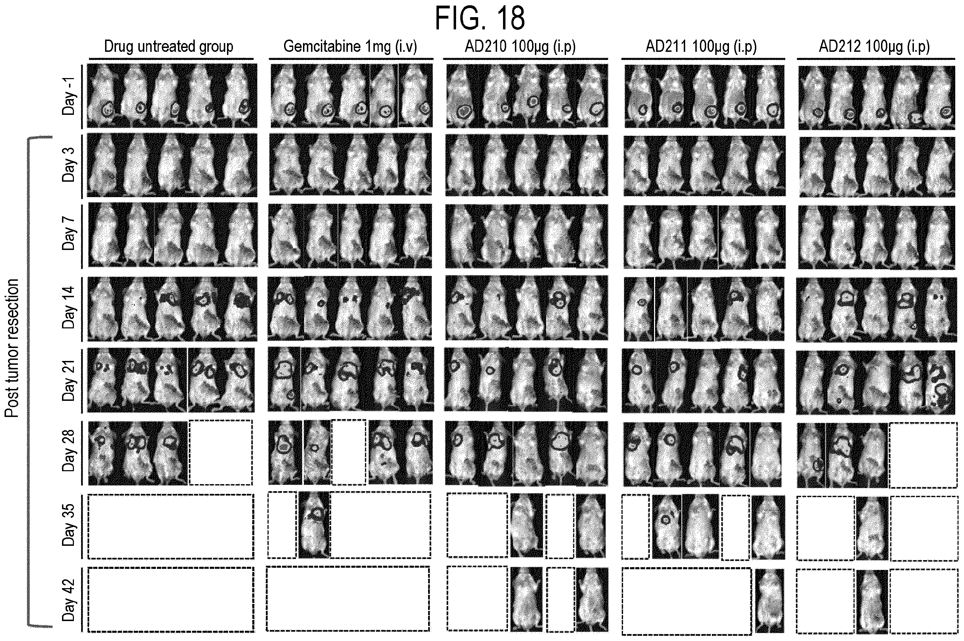

The results of metastasis and recurrence inhibition in the tumor resection model experiment are as shown in FIG. 18. The untreated group perished 35 days post-tumor resection, and the positive control gemcitabine-treated group perished due to metastasis by 42 days.

The test substances, AD210, AD211, and AD212, are novel compounds wherein gemcitabine is conjugated to the human serum albumin (HSA)- and K-RAS G12D-targeting nucleic acid ligand or substituted for some of the sequence of the human serum albumin (HSA)- and K-RAS G12D-targeting nucleic acid ligand. Each of the groups treated with the test substances had some individuals surviving until 42 days.

As of 42 days post-tumor resection, the AD210 100µg-administered group showed a 40% survival rate with 3 out of 5 mice deceased, and both the AD211 and AD212 100µg-administered groups had a 20% survival rate with 4 out of 5 mice deceased.

Particularly, 42 days post-tumor resection, the AD210 100µg-administered group demonstrated superior anti-metastasis and anti-recurrence effects, even though its dosage was approximately one-third of the AD005 300µg-administered group in the results of 7-3-3.

## Claims

1. A pharmaceutical composition for prevention or treatment of triple-negative breast cancer, the composition comprising a non-natural nucleic acid ligand or a pharmaceutical acceptable sale thereof that binds to human serum albumin (HSA) and K-RAS G12D.

2. The pharmaceutical composition of claim 1, wherein the non-natural nucleic acid ligand comprises the nucleic acid sequence of SEQ ID NO:
11(GNA6AAG6CCGGA6GGCAGC666A6GC) [wherein, the second nucleic acid N is cystidine and the portion marked as "6" is 5-[N-(1-naphthylmethyl) carboxamide]-2'-deoxyuridine (Nap-dU)].

3. The pharmaceutical composition of claim 1, wherein the composition comprises at least one of the non-natural nucleic acid ligands.

4. The pharmaceutical composition of claim 2, wherein the non-natural nucleic acid ligand comprising the nucleic acid sequence of SEQ ID NO: 11 comprises a nucleic acid sequence selected from SEQ ID NOS: 1 to 11.

5. The pharmaceutical composition of claim 1, wherein the triple-negative breast is primary or metastatic.

6. The pharmaceutical composition of claim 1, wherein the triple-negative breast cancer comprises cancer cells with a K-RAS mutant protein.

7. The pharmaceutical composition of claim 6, wherein the triple-negative breast cancer comprises cancer cells with a K-RAS G12D mutant protein.

8. The pharmaceutical composition of claim 1, wherein the pharmaceutical composition is administered intravenously, intramuscularly, intraarterially, intraperitoneally, intranasally, intravaginally, intravesically, intradermally, transdermally, topically, or subcutaneously to a subject in need thereof.

9. The pharmaceutical composition of claim 1, wherein the pharmaceutical composition is administered after surgical resection of a triple-negative breast cancer tissue.

10. The pharmaceutical composition of claim 1, wherein after surgical resection of a triple-negative breast cancer tissue, the pharmaceutical composition is administered into the resected site.

11. Non-natural nucleic acid ligand, comprising a nucleic acid sequence selected from SEQ ID NOS: 8 to 11 and binding to human serum albumin (HAS) and K-RAS G12D.

12. The non-natural nucleic acid ligand of claim 11, comprising a nucleic acid sequence selected from SEQ ID NOS: 8 to 11.

13. A diagnostic composition comprising the non-natural nucleic acid ligand of claim 11.

14. A contrast agent comprising the non-natural nucleic acid ligand of claim 11.

15. A radiopharmaceutical comprising the non-natural nucleic acid ligand of claim 11.

16. A composition for cancer diagnosis, comprising the non-natural nucleic acid ligand of claim 11.
